# EUROPEAN PATENT APPLICATION

(11) **EP 1 617 361 A1**
(43) Date of publication of application: **18.01.2006**
(21) Application number: 04726625.9
(22) Date of filing: 08.04.2004
(51) Int. Cl.: G06Q 10/00

(54) **AT-HOME MEDICAL EXAMINATION SYSTEM AND AT-HOME MEDICAL EXAMINATION METHOD**

(30) Priority: 11.04.2003 JP 2003108057
(71) Applicant: Ginganet Corporation, Osaka-shi, Osaka 556-0017 (JP)
(72) Inventor: SAHASHI, Nozomu, Kishiwada-shi, Osaka 5960045 (JP)
(74) Representative: Zimmermann, Tankred Klaus
(86) International application number: PCT/JP2004/005072
(87) International publication number: WO 2004/092991

(57) **Abstract**

An at-home medical consulting service having an environment similar to when a patient actually visits a hospital is provided, wherein a sudden deterioration in the patient's condition can be dealt with even when the patient terminal has not been connected to the doctor terminal and the patient's anxiety for the sickness and sense of isolation can be eliminated without increasing the burden on a doctor. An at-home medical consultation system (100) includes a reception server (110), a communication server (120), a mail server (130), an administration server (140), doctor terminals (161, ---), a nurse terminal (180), a waiting room terminal (175), a reception terminal (170) and a pharmacy terminal (190), and is connected to patient terminals (301, ---) via a communication line (200) to provide an at-home medical consultation service by videophone. The communication server (120) includes a destination table (122) and provides a virtual consultation room, a virtual waiting room, a virtual nursing room and a virtual individual conversation room depending upon the object. The patient terminal includes a nurse call button (g) and a medical examination sensor (h) such that the nurse terminal can be called in case the patient's condition has deteriorated suddenly and vitals of the patient can be monitored remotely.

## Description

### Technical Field

This invention relates to an at-home medical consultation system which enables a patient to consult a doctor from home over a videophone.

### Background Art

Due to the development of communication technology in recent years, it is becoming possible for persons at a distance from each other to hold a conversation on a practicable level using a videophone. Also in the medical field, attempts have been made to put an at-home medical consultation system to a practical use to enable a patient to receive a medical consultation service at home by connecting to a doctor terminal at a distance using a videophone.

A conventional at-home medical consultation system is disclosed in, for example, JP-A-11-89802 in which a doctor terminal and a patient terminal are connected together through a digital communication network, and a conversation is held between the doctor terminal and the patient terminal over a videophone to enable the patient to consult the doctor from home. In this system, sensors for measuring the patient's body temperature, heart beat, blood pressure, etc. are connected to the patient terminal. These sensors are attached to the patient's body, the values measured by the sensors are displayed on the doctor terminal, and the doctor properly examines the patient based upon these values and the expression and motion of the patient.

The above-described at-home medical consultation system enables the patient to consult the doctor from home, such that a person living in a doctorless village or in some remote place such as a foreign country, or an invalid or bedridden aged person may easily receive medical service.

Furthermore, the widespread use of such at-home medical consultation system enables the patient to receive a greatly increased freedom for medical consultation so that an accident that results from passing-around of, for example, an infantile patient, which is a problem arousing in recent years, can be prevented.

According to the conventional at-home medical consultation system, however, the patient terminal is disconnected from the doctor terminal except when the patient consults the doctor and the patient must wait for access from the doctor terminal for medical consultation, so it has a problem in which the situation cannot be dealt with when the patient's condition has deteriorated suddenly during that period.

When actually visiting a hospital, a patient's anxiety for sickness or sense of isolation can be eliminated by talking to other patients and exchanging information with each other while waiting in a waiting room of a hospital or by consulting a nurse. When consulting at home, however, the patient is unable to exchange information with other patients or to consult the nurse, except to talk to the doctor by connecting to the doctor terminal. This causes a problem in which the patient must spend the days dealing with the anxiety for the sickness and sense of isolation.

This is a serious problem, particularly, for the elderly persons living alone or for the bedridden patients.

Therefore, a principal object of the present invention is to provide an at-home medical consulting service in which a sudden deterioration in the patient's condition can be dealt with, even when the patient terminal is not connected to the doctor terminal, and the patient's anxiety for the sickness and sense of isolation can be eliminated without increasing the burden on a doctor.

### Disclosure of the Invention

An at-home medical consultation system described in claim 1 is a system for providing an at-home medical consulting service by connecting a patient terminal having a videophone function to a doctor terminal having a videophone function through a communication line, which includes a reception server for accepting a request for medical consultation from the patient terminal, and a communication server for providing a bidirectional communication function of video and audio by connecting the patient terminal whose request for medical consultation has been accepted to the doctor terminal, wherein the communication server includes a function for setting a virtual waiting room for providing a conversation service by video and audio among the patient terminals, and the reception server includes a function for connecting the patient terminal whose request for medical consultation has been accepted to the virtual waiting room that has been set.

With this configuration, the reception server connects the patient terminal whose request for medical consultation has been accepted to the virtual waiting room that has been set by the communication server, and the communication server provides a conversation service over the videophones among the patient terminals connected to the virtual waiting room, such that the patient can receive assistance from other patients who have been connected to the virtual waiting room even when the patient's condition deteriorates suddenly before consulting the doctor by connecting the patient terminal to the doctor terminal and the patient's anxiety for the sickness and sense of isolation are eliminated by exchanging information among the patients connected to the virtual waiting room. Thus, an at-home medical consulting service having an environment that is similar to when the patient actually visits the hospital is provided.

An at-home medical consultation system described in claim 2 is the at-home medical consultation system described in claim 1, wherein the communication server includes a function for connecting a waiting room terminal having a videophone function installed in a waiting room of a hospital to the virtual waiting room that has been set.

With this configuration, the communication server provides a conversation service over the videophones between the patient terminal connected to the virtual waiting room and the waiting room terminal installed in the waiting room of the hospital, such that the patient can receive assistance from other patients who are in the waiting room of the hospital, even when the patient's condition deteriorates suddenly before consulting the doctor by connecting the patient terminal to the doctor terminal and patient's anxiety for the sickness and sense of isolation are eliminated by exchanging information among the patients in the waiting room of the hospital. Thus, an at-home medical consulting service having an environment similar to when the patient actually visits the hospital is provided.

An at-home medical consultation system described in claim 3 is the at-home medical consultation system described in claim 1 or 2, wherein the reception server includes a function for selecting a callee terminal connected to the virtual waiting room based on a location on a screen specified by a pointing device at the caller terminal connected-to the virtual waiting room, and the communication server includes a function for providing a conversation service by video and audio by separately connecting the caller terminal to the callee terminal.

With this configuration, the reception server selects the callee terminal of the patient terminal specified by the pointing device at the caller terminal, and the communication server provides an individual conversation service over the videophones between the caller terminal and the callee terminal, such that private counseling with a particular patient can be performed. Thus, an at-home medical consulting service having an environment similar to when the patient actually visits the hospital is provided.

An at-home medical consultation system described in claim 4 is the at-home medical consultation system described in any one of claims 1 to 3, wherein the reception server includes a function for accepting a nurse call from the patient terminal and notifying a nurse terminal having a videophone function of the nurse call, and the communication server includes a function for providing a conversation service by video and audio by connecting the patient terminal whose nurse call has been accepted to the notified nurse terminal.

With this configuration, the reception server accepts a nurse call from the patient terminal and notifies the nurse terminal of the nurse call, and the communication server provides an individual conversation service over the videophones between the patient terminal whose nurse call has been accepted and the notified nurse terminal, so that the patient at home can ask the nurse for treatment when the condition has deteriorated suddenly or consult the nurse about anxiety for the sickness and the like. Thus, an at-home medical consulting service having an environment similar to when the patient actually visits the hospital is provided.

An at-home medical consultation system described in claim 5 is the at-home medical consultation system described in claim 4, wherein the patient terminal includes a nurse call button for transmitting a nurse call to the reception server, and includes a function for automatically logging into the reception server when the nurse call button is pressed to transmit the nurse call.

With this configuration, upon pressing a nurse call button provided for the patient terminal, the patient terminal automatically logs into the reception server, such that the patient can call the nurse terminal without going through the log-in procedure even when the condition has deteriorated suddenly before log-in. Thus, a quick at-home medical consulting service is provided even in case of emergency.

An at-home medical consultation system described in claim 6 is the at-home medical consultation system described in claim 4 or 5, wherein the communication server includes a function for setting a virtual nursing room which is connected the patient terminal used by a person who needs nursing, and the reception server includes a function for connecting the patient terminal used by the person who needs nursing to the virtual nursing room upon a request from the patient terminal, the doctor terminal or the nurse terminal. The communication server includes a function for transmitting the video and audio of the patient terminals connected to the virtual nursing room to the doctor terminal and/or the nurse terminal upon a request from the doctor terminal and/or the nurse terminal.

With this configuration, the reception server connects the patient terminal used by the person who needs nursing to the virtual nursing room set by the communication server upon a request from the patient terminal, the doctor terminal or the nurse terminal, and the communication server transmits the video and audio of the patient terminals connected to the virtual nursing room upon a request from the doctor terminal and/or the nurse terminal, such that the patient at home who must be monitored for a sudden deterioration in the condition can be checked regularly from the doctor terminal and/or from the nurse terminal. Thus, an at-home medical consulting service having an environment similar to when the patient is actually hospitalized is provided.

An at-home medical consultation system described in claim 7 is the at-home consultation system described in claim 6, wherein the reception server includes a function for selecting a patient terminal connected to the virtual nursing room based on a location on a screen specified by the pointing device at the doctor terminal and/or the nurse terminal, and the communication server includes a function for providing a conversation service by video and audio by separately connecting the doctor terminal and/or the nurse terminal to the selected patient terminal.

With this configuration, the reception server selects the patient terminal specified by the pointing device at the doctor terminal and/or the nurse terminal, and the communication server provides an individual conversation service over the videophones between the doctor terminal and/or the nurse terminal and the selected patient terminal, such that the doctor and/or the nurse can more clearly understand the sudden deterioration in the patient's conditions. Thus, an at-home medical consulting service having an environment similar to when the patient actually visits the hospital is provided.

An at-home medical consultation system described in claim 8 is the at-home medical consultation system described in claim 6 or 7, wherein the patient terminal includes medical examination sensors for collecting the data necessary for consulting the patient, and includes a function for obtaining the data from the medical examination sensors and transmitting the data to the communication server. The communication server includes a function for receiving the transmitted data from the medical examination sensors and transmitting the data to the doctor terminal and/or the nurse terminal. The doctor terminal and/or the nurse terminal include a function for receiving the transmitted data from the medical examination sensors and displaying them.

With this configuration, the data from the medical examination sensors provided at the patient terminal can be confirmed from the doctor terminal and/or the nurse terminal, such that the condition of the patient at home can be more accurately determined. Thus, an at-home medical consulting service having an environment similar to when the patient actually visits the hospital is provided.

The medical examination sensors preferably include, for example, vital sensors used for measuring the body temperature, heart beat, blood pressure, degree of oxygen saturation, electrocardiogram and so on, or a stethoscope used for stethoscoping the heart sound or the breath sound such that the patient can wear its sensor head by himself. The doctor may visit in advance to mount the sensor head and remotely take measurements at the time of medical consultation service at home.

An at-home medical consultation system described in claim 9 is the at-home consultation system described in claim 8, wherein the patient terminal includes a function for transmitting a portion or all of the signals of the medical examination sensors as audio signals.

With this configuration, for the sensors, such as a stethoscope, with which the doctor directly hears the sound that is detected for examination, the sensor signals are transmitted from the patient terminal as audio signals of the videophone, such that the sensor signals are directly heard via a headset for conversation at the doctor terminal. Thus, an at-home medical consulting service having an environment similar to when the patient actually visits the hospital is provided.

An at-home medical consultation system described in claim 10 is a system for providing an at-home medical consulting service by connecting a patient terminal having a videophone function to a doctor terminal having a videophone function through a communication line, and includes a reception server for accepting a request for medical consultation from the patient terminal, and a communication server for providing a bidirectional communication function of video and audio by connecting the patient terminal whose request for medical consultation has been accepted to the doctor terminal, wherein the communication server includes a function for setting a virtual nursing room to which the patient terminal used by a person who needs nursing is connected. The reception server includes a function for connecting the patient terminal used by the person who needs nursing to the virtual nursing room upon a request from the patient terminal, the doctor terminal or the nurse terminal having the videophone function. The communication server includes a function for transmitting video and audio of the patient terminals connected to the virtual nursing room to the doctor terminal and/or the nurse terminal upon a request from the doctor terminal and/or the nurse terminal.

With this configuration, the reception server connects the patient terminal used by the person who needs nursing to the virtual nursing room that has been set upon a request from the patient terminal, doctor terminal or nurse terminal, and the communication server transmits video and audio of the patient terminals connected to the virtual nursing room upon a request from the doctor terminal and/or the nurse terminal, such that the patient at home who must be monitored for a sudden deterioration in condition can be checked regularly from the doctor terminal and/or the nurse terminal. Thus, an at-home medical consulting service having an environment similar to when the patient is actually hospitalized is provided.

An at-home consultation system described in claim 11 is the at-home consultation system described in claim 10, wherein the reception server includes a function for selecting the patient terminal connected to the virtual nursing room based on a location on a screen specified by a pointing device at the doctor terminal and/or the nurse terminal, and the communication server includes a function for providing a conversation service by video and audio by separately connecting the doctor terminal and/or the nurse terminal to the selected patient terminal.

With this configuration, the reception server selects the patient terminal specified by the pointing device at the doctor terminal and/or the nurse terminal, and the communication server provides an individual conversation service over the videophones between the doctor terminal and/or the nurse terminal and the selected patient terminal, such that the doctor and/or the nurse can more clearly understand a sudden deterioration in the patient's condition. Thus, an at-home medical consulting service having an environment similar to when the patient actually visits the hospital is provided.

An at-home consultation system described in claim 12 is the at-home consultation system described in claim 10 or 11, wherein the reception server includes a function for accepting the nurse call from the patient terminal and notifying the nurse terminal of the nurse call, and the communication server includes a function for providing a conversation service by video and audio by connecting the patient terminal whose nurse call has been accepted to the notified nurse terminal.

With this configuration, the reception server receives the nurse call from the patient terminal and notifies the nurse terminal of the nurse call, and the communication server provides an individual conversation service over the videophones between the patient terminal whose nurse call has been accepted and the notified nurse terminal, such that the patient at home can ask the nurse for treatment when the condition has deteriorated suddenly or consult the nurse about anxiety for the sickness and the like. Thus, an at-home medical consulting service having an environment similar to when the patient actually visits the hospital is provided.

An at-home medical consultation system described in claim 13 is the at-home medical consultation system described in claim 12, wherein the patient terminal includes a nurse call button for transmitting a nurse call to the reception server, and includes a function for automatically logging into the reception server when the nurse call button is pressed to transmit the nurse call.

With this configuration, upon pressing a nurse call button provided for the patient terminal, the patient terminal can automatically log into the reception server, such that the patient can call the nurse terminal without going through log-in procedure even when the condition has deteriorated suddenly before log-in. Thus, a quick at-home medical consulting service is provided even in case of emergency.

An at-home medical consultation system described in claim 14 is the at-home medical consultation system described in any one of claims 10 to 13, wherein the patient terminal includes medical examination sensors for collecting the data necessary for consulting the patient, and includes a function for obtaining the data from the medical examination sensors and transmitting the data to the communication server. The communication server includes a function for receiving the transmitted data from the medical examination sensors and transmitting the data to the doctor terminal and/or the nurse terminal, and the doctor terminal and/or the nurse terminal include a function for receiving the transmitted data from the medical examination sensors and displaying them.

With this configuration, the data from the medical examination sensors provided at the patient terminal can be confirmed from the doctor terminal and/or the nurse terminal such that the condition of the patient at home can be more clearly understood. Thus, an at-home medical consulting service having an environment similar to when the patient actually visits the hospital is provided.

The medical examination sensors preferably include, for example, vital sensors used for measuring the body temperature, heart beat, blood pressure, degree of oxygen saturation, electrocardiogram and so on, or a stethoscope used for stethoscoping the heart sound or the breath sound such that the patient can wear its sensor head by himself. The doctor may visit in advance to mount the sensor head and remotely take a measurement at the time of medical consultation service at home.

An at-home medical consultation system described in claim 15 is the at-home medical consultation system described in claim 14, wherein the patient terminal includes a function for transmitting a portion or all of the signals of the medical examination sensors as voice signals.

With this configuration, as for the sensors like a stethoscope with which the doctor directly hears the sound that is detected for examination, the sensor signals are transmitted from the patient terminal as voice signals of the videophone, such that the sensor signals can be directly heard via using a headset for conversation at the doctor terminal. Thus, an at-home medical consulting service having an environment similar to when the patient actually visits the hospital is provided.

An at-home medical consultation system described in claim 16 is a system for providing an at-home medical consulting service by connecting the patient terminal having a videophone function to the doctor terminal having a videophone function through a communication line, and includes a reception server for accepting a request for medical consultation from the patient terminal, and a communication server for providing a bidirectional communication function of video and audio by connecting the patient terminal whose request for medical consultation has been accepted to the doctor terminal, wherein the reception server includes a function for accepting a nurse call from the patient terminal and notifying the nurse terminal having the videophone function of the nurse call, and the communication server includes a function for providing a conversation service by video and audio by connecting the patient terminal whose nurse call has been accepted to the notified nurse terminal.

With this configuration, the reception server accepts a nurse call from the patient terminal and notifies the nurse terminal of the nurse call, and the communication server provides an individual conversation service over the videophones between the patient terminal whose nurse call has been accepted and the notified nurse terminal, such that the patient at home can ask the nurse for treatment when the condition of the patient has deteriorated suddenly or consult the nurse about anxiety for the sickness and the like. Thus, an at-home medical consulting service having an environment similar to when the patient actually visits the hospital is provided.

An at-home medical consultation system described in claim 17 is the at-home medical consultation system described in claim 16, wherein the patient terminal includes a nurse call button for transmitting a nurse call to the reception server, and includes a function for automatically logging into the reception server when the nurse call button is pressed to transmit the nurse call.

With this configuration, upon pressing a nurse call button provided for the patient terminal, the patient terminal can automatically log into the reception server, such that the patient can call the nurse terminal without going through log-in procedure even when the condition has deteriorated suddenly before log-in. Thus, a quick at-home medical consulting service can be provided even in case of emergency.

An at-home medical consultation system described in claim 18 is the at-home medical consultation system described in any one of claims 1 to 17, wherein the reception server includes a function for accepting a request for medication from the patient terminal, a function for requesting the doctor terminal used by a doctor in charge of the patient whose request for medication has been accepted to confirm the medication, and a function for delivering a prescription issued by the doctor terminal in response to the request to a pharmacy terminal used by a pharmacy that offers home delivery service of medicine.

With this configuration, the patient at home can ask the doctor in charge for medication without actually visiting the hospital and to receive the medicine by the home delivery service.

An at-home medical consultation system described in claim 19 is the at-home medical consultation system described in any one of claims 1 to 18, wherein the reception server includes a function for accepting a request for counseling from the patient terminal, a function for receiving counseling sentences from the patient terminal whose request for counseling has been accepted, a function for transmitting the received counseling sentences to the doctor terminal used by a doctor in charge, a function for receiving reply sentences from the doctor terminal in response to the transmitted counseling sentences, and a function for transmitting the received reply sentences to the patient terminal whose request for counseling has been accepted.

With this configuration, the patient at home can ask the doctor in charge for the counseling concerning the medical treatment without actually visiting hospital, such that the patient can eliminate anxiety for the sickness or consult the doctor about the therapy without increasing a burden on the doctor.

An at-home medical consultation system described in claim 20 is the at-home medical consultation system described in any one of claims 1 to 19, wherein the reception server includes a function for accepting a reservation for visit consultation from the patient terminal.

With this configuration, the patient at home can make a reservation for visit consultation without actually visiting the hospital, such that the patient can consult the doctor in charge efficiently by minimizing the waiting time in the hospital.

An at-home medical consultation method described in claim 21 is a method of providing an at-home medical consulting service by connecting a patient terminal having a videophone function to a doctor terminal having a videophone function through a communication line, and includes a step of accepting a request for medical consultation from the patient terminal; and a step of providing a bidirectional communication function of video and audio by connecting the patient terminal whose request for medical consultation has been accepted to the doctor terminal, wherein the step of providing the bidirectional communication of video and audio includes a step of setting a virtual waiting room for providing a conversation service by video and audio among the accepted patient terminals, and the step of accepting the request for medical consultation includes a step of connecting the patient terminal whose request for medical consultation has been accepted to the virtual waiting room that has been set.

With this configuration, the step of accepting the request for medical consultation connects the patient terminal whose request for medical consultation has been accepted to the virtual waiting room set by the step of providing the bidirectional communication of video and audio, and the step of providing the bidirectional communication of video and audio provides a conversation service over videophones among the patient terminals that have been connected to the virtual waiting room, such that the patient can receive assistance from other patients who have been connected to the virtual waiting room, even when the patient's condition has deteriorated suddenly before consulting the doctor by connecting the patient terminal to the doctor terminal and the patient's anxiety for the sickness and sense of isolation are eliminated by exchanging information among the patients connected to the virtual waiting room. Thus, an at-home medical consulting service having an environment similar to when the patient actually visits the hospital is provided.

An at-home medical consultation method described in claim 22 is the at-home medical consultation method described in claim 21, wherein the step of providing the bidirectional communication of video and audio includes a step of connecting a waiting room terminal having a videophone function installed in a waiting room of a hospital to the virtual waiting room that has been set.

With this configuration, the step of providing the bidirectional communication of video and audio provides a conversation service over the videophones between the patient terminal connected to the virtual waiting room and the waiting room terminal installed in the waiting room of the hospital, such that the patient can receive assistance from other patients who are in the waiting room of the hospital even when the patient's condition has deteriorated suddenly before consulting the doctor by connecting the patient terminal to the doctor terminal and the patient's anxiety for the sickness and sense of isolation can be eliminated by exchanging information among the patients in the waiting room of the hospital. Thus, an at-home medical consulting service having an environment similar to when the patient actually visits the hospital is provided.

An at-home medical consultation method described in claim 23 is the at-home medical consultation method described in claim 21 or 22, wherein the step of accepting the request for medical consultation includes a step of selecting a callee terminal connected to the virtual waiting room based on a location on a screen specified by a pointing device at the caller terminal connected to the virtual waiting room, and the step of providing the bidirectional communication of video and audio includes a step of providing a conversation service by video and audio by separately connecting the caller terminal to the callee terminal.

With this configuration, the step of accepting a request for medical consultation selects the callee terminal of the patient terminal specified by the pointing device at the caller terminal, and the step of providing the bidirectional communication of video and audio provides an individual conversation service over the videophones between the caller terminal and the callee terminal, such that the private counseling with the particular patient can be performed. Thus, an at-home medical consulting service having an environment similar to when the patient actually visits the hospital is provided.

An at-home medical consultation method described in claim 24 is the at-home medical consultation method described in any one of claims 21 to 23, wherein the step of accepting the request for medical consultation includes a step of accepting a nurse call from the patient terminal and notifying a nurse terminal having a videophone function of the nurse call, and the step of providing the bidirectional communication of video and audio includes a step of providing a conversation service by video and audio by connecting the patient terminal whose nurse call has been accepted to the notified nurse terminal.

With this configuration, the step for accepting the request for medical consultation accepts a nurse call from the patient terminal and notifies the nurse terminal of the nurse call, and the step of providing the bidirectional communication of video and audio provides an individual conversation service over the videophones between the patient terminal whose nurse call has been accepted and the notified nurse terminal, such that the patient at home can ask the nurse for treatment when the condition has deteriorated suddenly or consult the nurse about anxiety for the sickness and the like. Thus, an at-home medical consulting service having an environment similar to when the patient actually visits the hospital is provided.

An at-home medical consultation method described in claim 25 is the at-home medical consultation method described in claim 24, wherein the step of providing the bidirectional communication of video and audio includes a step of setting a virtual nursing room to which is connected the patient terminal used by a person who needs nursing, the step of accepting the request for medical consultation includes a step of connecting the patient terminal used by the person who needs nursing to the virtual nursing room upon a request from the patient terminal, the doctor terminal or the nurse terminal, and the step of providing the bidirectional communication of video and audio includes a step of transmitting the video and audio of the patient terminals connected to the virtual nursing room to the doctor terminal and/or the nurse terminal upon a request from the doctor terminal and/or the nurse terminal.

With this configuration, the step of accepting the request for medical consultation connects the patient terminal used by the person who needs nursing to the virtual nursing room set by the step of providing the bidirectional communication of video and audio upon a request from the patient terminal, the doctor terminal or the nurse terminal, and the step of providing the bidirectional communication of video and audio transmits the video and audio of the patient terminals connected to the virtual nursing room upon a request from the doctor terminal and/or the nurse terminal, such that the patient at home who must be monitored for a sudden deterioration in condition can be checked regularly from the doctor terminal and/or from the nurse terminal. Thus, an at-home medical consultation method having an environment similar to when the patient is actually hospitalized is provided.

An at-home medical consultation method described in claim 26 is the at-home medical consultation method described in claim 25, wherein the step of accepting the request for medical consultation includes a step of selecting a patient terminal connected to the virtual nursing room based on a location on a screen specified by the pointing device at the doctor terminal and/or the nurse terminal, and the step of providing the bidirectional communication of video and audio includes a step of providing a conversation service by video and audio by separately connecting the doctor terminal and/or the nurse terminal to the selected patient terminal.

With this configuration, the step of accepting the request for medical consultation selects the patient terminal specified by the pointing device at the doctor terminal and/or the nurse terminal, and the step of providing the bidirectional communication of video and audio provides an individual conversation service over the videophones between the doctor terminal and/or the nurse terminal and the selected patient terminal, such that the doctor and/or the nurse can more clearly understand a sudden deterioration in the patient's condition. Thus, an at-home medical consulting service having an environment similar to when the patient actually visits the hospital is provided.

An at-home medical consultation method described in claim 27 is the at-home medical consultation method described in claim 25 or 26, wherein the step of providing the bidirectional communication of video and audio includes a step of receiving the data obtained from the medical examination sensors provided at the patient terminal and transmitting them to the doctor terminal and/or the nurse terminal.

With this configuration, the data of the medical examination sensors provided at the patient terminal can be confirmed from the doctor terminal and/or the nurse terminal such that the condition of the patient at home can be more clearly determined. Thus, an at-home medical consulting service having an environment similar to when the patient actually visits the hospital is provided.

The medical examination sensors preferably include, for example, vital sensors used for measuring the body temperature, heart beat, blood pressure, degree of oxygen saturation, electrocardiogram and so on, or a stethoscope used for stethoscoping the heart sound and breath sound, such that the patient can wear its sensor head by himself. The doctor may visit in advance to mount the sensor head and remotely take a measurement at the time of medical consultation service at home.

An at-home medical consultation method described in claim 28 is a method of providing an at-home medical consulting service by connecting a patient terminal having a videophone function to a doctor terminal having a videophone function through a communication line, and includes a step of accepting a request for medical consultation from the patient terminal, and a step of providing a bidirectional communication function of video and audio by connecting the patient terminal whose request for medical consultation has been accepted to the doctor terminal, wherein the step of providing the bidirectional communication of video and audio includes a step of setting a virtual nursing room to which is connected the patient terminal used by the person who needs nursing, the step of accepting the request for medical consultation includes a step of connecting the patient terminal used by the person who needs nursing to the virtual nursing room upon a request from the patient terminal, the doctor terminal or the nurse terminal having the videophone function, and the step of providing the bidirectional communication of video and audio includes a step of synthesizing the video and audio of the patient terminal connected to the virtual nursing room upon a request from the doctor terminal and/or the nurse terminal and delivering them to the doctor terminal and/or the nurse terminal.

With this configuration, the step of accepting the request for medical consultation connects the patient terminal used by the person who needs nursing to the virtual nursing room set by the step of providing the bidirectional communication of video and audio upon a request from the patient terminal, the doctor terminal or the nurse terminal, and the step of providing the bidirectional communication of video and audio transmits the video and audio of the patient terminals connected to the virtual nursing room upon a request from the doctor terminal and/or the nurse terminal, such that the patient at home who must be monitored for a sudden deterioration in condition can be monitored and checked regularly from the doctor terminal and/or the nurse terminal. Thus, an at-home medical consulting service having an environment similar to when the patient is actually hospitalized is provided.

An at-home medical consultation method described in claim 29 is the at-home medical consultation method described in claim 28, wherein the step of accepting the request for medical consultation includes a step of selecting the patient terminal connected to the virtual nursing room based on a location on a screen specified by a pointing device at the doctor terminal and/or the nurse terminal, and the step of providing the bidirectional communication of video and audio includes a step of providing a conversation service by video and audio by separately connecting the doctor terminal and/or the nurse terminal to the selected patient terminal.

With this configuration, the step of accepting the request for medical consultation selects the patient terminal specified by the pointing device at the doctor terminal and/or the nurse terminal, and the step of providing the bidirectional communication of video and audio provides an individual conversation service over the videophones between the doctor terminal and/or the nurse terminal and the selected patient terminal, such that the doctor and/or the nurse can more clearly understand a sudden deterioration in the patient's condition. Thus, an at-home medical consulting service having an environment similar to when the patient actually visits the hospital is provided.

An at-home medical consultation method described in claim 30 is the at-home medical consultation method described in claim 28 or 29, wherein the step of accepting a request for medical consultation includes a step of accepting a nurse call from the patient terminal and notifying the nurse terminal of the nurse call, and the step of providing the bidirectional communication of video and audio includes a step of providing a conversation service by video and audio by connecting the patient terminal whose nurse call has been accepted to the notified nurse terminal.

With this configuration, the step of accepting the request for medical consultation accepts the nurse call from the patient terminal and notifies the nurse terminal of the nurse call, and the step of providing the bidirectional communication of video and audio provides an individual conversation service over the videophones between the patient terminal whose nurse call has been accepted and the notified nurse terminal, such that the patient at home can ask the nurse for treatment when the condition has deteriorated suddenly or consult the nurse about anxiety for the sickness. Thus, an at-home medical consulting service having an environment similar to when the patient actually visits the hospital is provided.

An at-home medical consultation method described in claim 31 is the at-home medical consultation method described in any one of claims 28 to 30, wherein the step of providing the bidirectional communication of video and audio includes a step of receiving the data obtained by the medical examination sensors provided at the patient terminal and transmitting them to the doctor terminal and/or the nurse terminal.

With this configuration, the data of the medical examination sensors provided at the patient terminal can be confirmed from the doctor terminal and/or the nurse terminal, such that the condition of the patient at home can be accurately determined. Thus, an at-home medical consulting service having an environment similar to when the patient actually visits the hospital is provided.

The medical examination sensors preferably include, for example, vital sensors used for measuring, the body temperature, heart beat, blood pressure, degree of oxygen saturation, electrocardiogram and so on, or a stethoscope used for stethoscoping the heart sound or the breath sound, such that the patient can wear its sensor head by himself. The doctor may visit in advance to mount the sensor head and remotely take a measurement at the time of medical consultation service at home.

An at-home medical consulting method described in claim 32 is a method of providing an at-home medical consulting service by connecting a patient terminal having a videophone function to a doctor terminal having a videophone function through a communication line, and includes a step of accepting a request for medical consultation from the patient terminal, and a step of providing a bidirectional communication function of video and audio by connecting the patient terminal whose request for medical consultation has been accepted to the doctor terminal, wherein the step of accepting the request for medical consultation includes a step of accepting a nurse call from the patient terminal and notifying a nurse terminal having a videophone function of the nurse call, and the step of providing the bidirectional communication of video and audio includes a step of providing a conversation service by video and audio by connecting the patient terminal whose nurse call has been accepted to the notified nurse terminal.

With this configuration, the step of accepting the request for medical consultation accepts the nurse call from the patient terminal and notifies the nurse terminal of the nurse call, and the step of providing the bidirectional communication of video and audio provides an individual conversation service over the videophones between the patient terminal whose nurse call has been accepted and the notified nurse terminal, such that the patient at home can ask the nurse for treatment when the condition has deteriorated suddenly or consult the nurse about anxiety for the sickness and the like. Thus, an at-home medical consulting service having an environment similar to when the patient actually visits the hospital is provided.

An at-home medical consultation method described in claim 33 is the at-home medical consultation method described in any one of claims 21 to 32, wherein the step of accepting a request for medical consultation includes a step of accepting a request for medication from the patient terminal, a step of requesting the doctor terminal used by the doctor in charge of the patient whose request for medication has been accepted to confirm the medication, and a step of delivering a prescription issued by the doctor terminal in response to the request to a pharmacy terminal used by a pharmacy that offers a home delivery service of medicine.

With this configuration, the patient at home can ask the doctor in charge for medication without actually visiting the hospital and to receive the medicine by the home delivery service.

An at-home medical consultation method described in claim 34 is the at-home medical consultation method described in any one of claims 21 to 33, wherein the step of accepting the request for medical consultation includes a step of accepting a request for counseling from the patient terminal, a step of receiving counseling sentences from the patient terminal whose request for counseling has been accepted, a step of transmitting the received counseling sentences to the doctor terminal used by a doctor in charge, a step of receiving reply sentences from the doctor terminal in response to the transmitted counseling sentences, and a step of transmitting the received reply sentences to the patient terminal whose request for counseling has been accepted.

With this configuration, the patient at home can ask the doctor in charge for counseling concerning the medical treatment without actually visiting the hospital, such that the patient can eliminate anxiety for the sickness or consult about the therapy without increasing a burden on the doctor.

An at-home medical consultation method described in claim 35 is the at-home medical consultation method described in any one of claims 21 to 34, wherein the step of accepting a request for medical consultation includes a step of accepting a reservation for visit consultation from the patient terminal.

With this configuration, the patient at home can make a reservation for visit consultation without actually visiting the hospital, such that the patient can consult the doctor in charge efficiently by minimizing the waiting time in the hospital.

An at-home medical consultation program described in claim 36 is for executing, by using a computer, the at-home medical consultation method described in any one of claims 21 to 35.

Upon executing the at-home medical consultation program by using a computer and by making a connection to the patient terminal through the communication line, the same action and effect as the action and effect exhibited by the at-home medical consultation method described in any one of claims 21 to 35 are exhibited.

The above objects as well as other objects, features and advantages of the present invention will become more obvious from the following detailed description of the embodiments of the invention in conjunction with the drawings.

### Brief Description of the Drawings

Fig. 1 is a system block diagram of an at-home medical consultation system according to an embodiment of the present invention;
Fig. 2 is a diagram illustrating an example of setting a destination table provided in a communication server in the at-home medical consultation system according to the embodiment of the present invention;
Fig. 3 is a diagram illustrating examples of medical examination sensors provided at a patient terminal in the at-home medical consultation system according to the embodiment of the present invention;
Fig. 4 is a flowchart (No. 1) illustrating a procedure of a patient side processing in a reception server in the at-home medical consultation system according to the embodiment of the present invention;
Fig. 5 is a flowchart (No. 2) illustrating the procedure of the patient side processing in the reception server in the at-home medical consultation system according to the embodiment of the present invention;
Fig. 6 is a flowchart (No. 3) illustrating the procedure of the patient side processing in the reception server in the at-home medical consultation system according to the embodiment of the present invention;
Fig. 7 is a flowchart (No. 4) illustrating the procedure of the patient side processing in the reception server in the at-home medical consultation system according to the embodiment of the present invention;
Fig. 8 is a flowchart (No. 5) illustrating the procedure of the patient side processing in the reception server in the at-home medical consultation system according to the embodiment of the present invention;
Fig. 9 is a flowchart (No. 6) illustrating the procedure of the patient side processing in the reception server in the at-home medical consultation system according to the embodiment of the present invention;
Fig. 10 is a flowchart (No. 7) illustrating the procedure of the patient side processing in the reception server in the at-home medical consultation system according to the embodiment of the present invention;
Fig. 11 is a flowchart (No. 8) illustrating the procedure of the patient side processing in the reception server in the at-home medical consultation system according to the embodiment of the present invention;
Fig. 12 is a flowchart (No. 9) illustrating the procedure of the patient side processing in the reception server in the at-home consultation system according to the embodiment of the present invention;
Fig. 13 is a flowchart (No. 1) illustrating a procedure of a nurse side processing in the reception server in the at-home medical consultation system according to the embodiment of the present invention;
Fig. 14 is a flowchart (No. 2) illustrating the procedure of the nurse side processing in the reception server in the at-home medical consultation system according to the embodiment of the present invention;
Fig. 15 is a flowchart (No. 3) illustrating the procedure of the nurse side treatment in the reception server in the at-home consultation system according to the embodiment of the present invention;
Fig. 16 is a flowchart (No. 4) illustrating the procedure of the nurse side processing in the reception server in the at-home medical consultation system according to the embodiment of the present invention;
Fig. 17 is a flowchart (No.5) illustrating the procedure of the nurse side processing in the reception server in the at-home medical consultation system according to the embodiment of the present invention;
Fig. 18 is a flowchart (No. 1) illustrating a procedure of a doctor side processing in the reception server in the at-home medical consultation system according to the embodiment of the present invention;
Fig. 19 is a flowchart (No.2) illustrating the procedure of the doctor side processing in the reception server in the at-home medical consultation system according to the embodiment of the present invention;
Fig. 20 is a flowchart (No. 3) illustrating the procedure of the doctor side processing in the reception server in the at-home medical consultation system according to the embodiment of the present invention;
Fig. 21 is a flowchart (No. 4) illustrating the procedure of the doctor side processing in the reception server in the at-home medical consultation system according to the embodiment of the present invention;
Fig. 22 is a flowchart (No. 5) illustrating the procedure of the doctor side processing in the reception server in the at-home medical consultation system according to the embodiment of the present invention;
Fig. 23 is a flowchart (No. 6) illustrating the procedure of the doctor side processing in the reception server in the at-home medical consultation system according to the embodiment of the present invention;
Fig. 24 is a diagram illustrating a patient reception screen displayed on a patient terminal;
Fig. 25 is a diagram illustrating a new registration screen displayed on the patient terminal;
Fig. 26 is a diagram illustrating a patient menu screen displayed on the patient terminal;
Fig. 27 is a diagram illustrating a first visit reception screen displayed on the patient terminal;
Fig. 28 is a diagram illustrating a medical consultation reception completion screen displayed on the patient terminal;
Fig. 29 is a diagram illustrating a waiting room screen displayed on the patient terminal;
Fig. 30 is a diagram illustrating a medical consultation guidance screen displayed on the patient terminal;
Fig. 31 is a diagram illustrating a medical consultation screen (patient side) displayed on the patient terminal;
Fig. 32 is a diagram illustrating a re-visit reception screen displayed on the patient terminal;
Fig. 33 is a diagram illustrating a medication reception screen displayed on the patient terminal;
Fig. 34 is a diagram illustrating a counseling reception screen displayed on the patient terminal;
Fig. 35 is a diagram illustrating a visit reservation screen displayed on the patient terminal;
Fig. 36 is a diagram illustrating a nursing reception screen displayed on the patient terminal;
Fig. 37 is a diagram illustrating a nursing room entrance screen displayed on the patient terminal;
Fig. 38 is a diagram illustrating a nurse menu screen displayed on a nurse terminal;
Fig. 39 is a diagram illustrating a medical consultation accepted person confirmation screen displayed on the nurse terminal;
Fig. 40 is a diagram illustrating a patient's file display screen displayed on the nurse terminal;
Fig. 41 is a diagram illustrating a nursing room confirmation screen displayed on the nurse terminal;
Fig. 42 is a diagram illustrating a nurse call reception screen displayed on the nurse terminal;
Fig. 43 is a diagram illustrating a doctor menu screen displayed on a doctor terminal;
Fig. 44 is a diagram illustrating a medical consultation screen (doctor side) displayed on the doctor terminal;
Fig. 45 is a diagram illustrating a counseling processing screen displayed on the doctor terminal;
Fig. 46 is a diagram illustrating a reply mail of counseling transmitted to the patient terminal; and
Fig. 47 is a diagram illustrating a medication processing screen displayed on the doctor terminal.

### Best Mode for Carrying Out the Invention

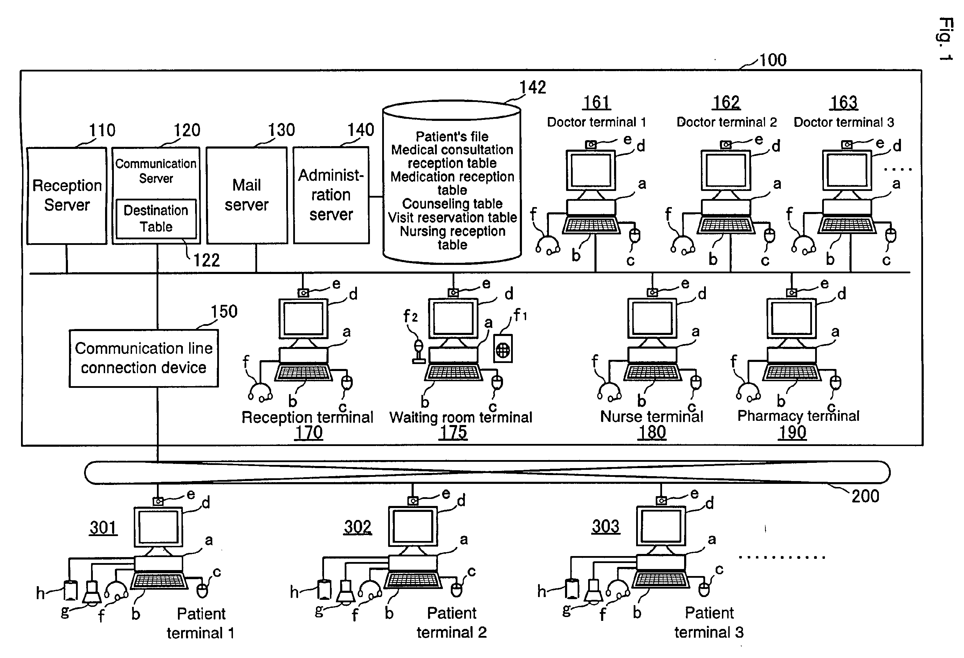

Fig. 1 is a system block diagram of an at-home medical consultation system according to an embodiment of the present invention, wherein reference numeral 100 represents an at-home medical consultation system in which a reception server 110, a communication server 120, a mail server 130, an administration server 140, doctor terminals 161, 162, 163... used by the doctors, a reception terminal 170 used by the receptionist of a hospital, a waiting room terminal 175 installed in a waiting room of the hospital, a nurse terminal 180 used by the nurses, a pharmacy terminal 190 used by the pharmacy which delivers the medicine and a communication line connection device 150 are connected together through a network (LAN) in the hospital, and are connected to the patient terminals 301, 302, 303... used by the patients through a communication line 200 when providing an at-home medical consultation service.

The reception server 110 is a WWW server that becomes a service center when this system is to be used. Various procedures can be taken for the at-home medical consultation service by accessing a predetermined page from the patient terminals 301, 302, 303..., doctor terminals 161, 162, 163..., and nurse terminal 180 etc. using a web browser.

The communication server 120 includes a destination table 122 for setting a terminal address of a terminal that is to be connected, connects the terminals set in the destination table, and provides a bidirectional communication function using multimedia information including video and audio. Therefore, the communication server 120 includes a function for connecting multiple terminals among the terminals corresponding to a videophone protocol used by the connected terminals.

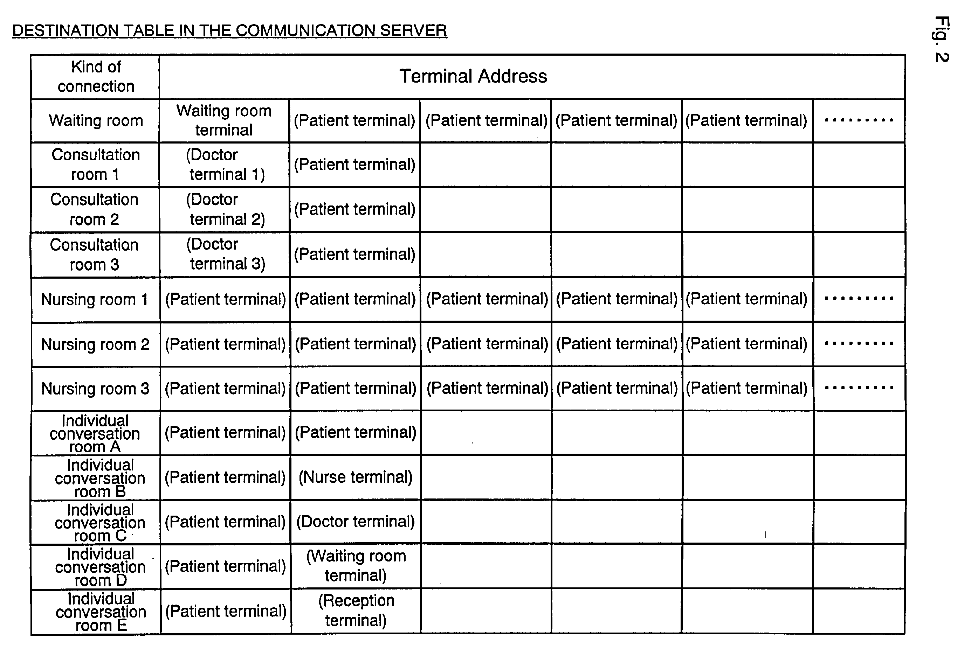

The communication server 120 supports a variety of connection modes depending upon the desired operation. The destination table 122 includes, as shown in Fig. 2, a virtual waiting room to which are connected the waiting room terminal 170 and the patient terminals that are being connected to the at-home consultation system, virtual consultation rooms 1, 2, 3..., each provided for the doctor terminals 161, 162, 163..., and to which the patient terminals who are to conduct an at-home medical consultation are connected, virtual nursing rooms 1, 2, 3..., to which the patient terminals used by patients who need nursing are connected, such as patients who must be monitored for a deterioration in their condition, patients living alone and bedridden patients, of which the conditions can be confirmed from the nurse terminal or the doctor terminal, and virtual individual conversation rooms A, B, C, D, E... capable of providing individual conversation functions, such as individual conversation between the patient terminals, between the patient terminal and the nurse terminal, between the patient terminal and the waiting room terminal, and between the patient terminal and the reception terminal. Conversations can be conducted simultaneously over the videophones within the processing capability of the communication server 120.

Here, the terminals are connected with each other through a computer network inclusive of the internet. It is therefore assumed that an IP address of a terminal that is to be connected is set to the destination table. In the case of, for example, an ISDN videophone to which the connected terminal makes a communication through an ISDN line, a telephone number of the terminal is set to the destination table.

The mail server 130 is used for sending a reply of medical counseling or for sending various notices to the patient terminal which is off line. The mail server needs not necessarily be provided in the system, and a mail server provided by a line connection provider may be used.

The administration server 140 is used for managing the operation of the hospital and includes a database 142 for storing a patient's file recording the history of medical consultation of a patient, a medical consultation reception table for registering a patient whose medical consultation has been accepted, a medication reception table for registering a patient for whose medication has been accepted, a counseling reception table for registering a patient whose counseling has been accepted, a visit reservation table for registering a patient whose visit has been accepted and a nursing reception table for registering a patient who must be monitored for a turn of condition.

This embodiment is not directed to a hospital that is dedicated to at-home medical consultation, but is directed to a hospital which accepts ordinary visits and hospitalization. Therefore, the administration server 140 deals with the ordinary outpatients, inpatients as well as patients at home. Therefore, the reception terminal accepts the outpatients and inpatients, notifies the doctor terminals and the nurse terminal of the reception data, instructs the pharmacy terminal for medication in accordance with a prescription from the doctor terminal, and calculates the fees for medical treatment based on the data from the doctor terminal and the pharmacy terminal. However the present invention is directed to the at-home medical consultation, the detailed description of the administration related to the ordinary visit or hospitalization is omitted.

The doctor terminals 161, 162, 163..., reception terminal 170, nurse terminal 180, and pharmacy terminal 190 are each provided with a computer (a) equipped with an video input interface/audio input-output interface/network connection device, a keyboard (b)/mouse (c) for inputting commands and messages, a monitor screen (d) for displaying video, a TV camera (e) for obtaining the user's video, and a headset (f) for inputting/outputting the user's audio. In the computer (a), a video/audio communication program for obtaining the video and audio of the user and for transmitting the video and audio to the communication server 120, and for outputting the video and audio transmitted from the communication server 120 to the monitor screen (d) and to the headset (f), and a web browser for accessing the reception server 110 are installed.

The waiting room terminal 175 is provided with a computer (a), a keyboard (b), a mouse (c), a monitoring screen (d), and a TV camera (e), similar to the doctor terminals. Instead of a headset (f), however, a speaker (f1) and a microphone (f2) are provided, such that the patient can conduct a conversation with people in the waiting room of the hospital.

The communication line 200 may be compatible with any type of communication line, such as a wired computer network, a wireless computer network, a telephone line, a satellite line or internet. Here describes a case using the internet. Therefore, the communication line connection device 150 uses an internet rooter for connecting to the internet.

On the other hand, the patient terminals 301, 302, 303... are each provided with a computer (a) having an video input interface/audio input-output interface/network connection device, a keyboard (b)/mouse (c) for inputting commands and messages, a monitor screen (d) for displaying image, a TV camera (e) for obtaining the patient's video, and a headset (f) for inputting/outputting the patient's audio, similar to the doctor terminals. Further, a nurse call button (g) for calling the nurse terminal from the patient terminal and a medical examination sensor (h) for vital measurement of the patient are provided. In the computer (f), a video/audio communication program for obtaining the video and audio of the patient, for transmitting the video and audio to the communication server 120, and for outputting the video and audio transmitted from the communication server 120 to the monitor screen (d) and to the headset (f), and a web browser for accessing the reception server 110 are installed.

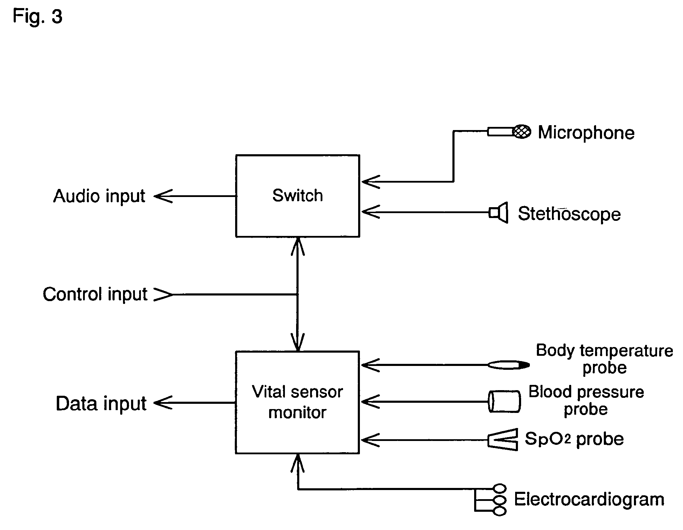

The medical examination sensor (h) includes, as shown in Fig. 3, a stethoscope for hearing the heart sound of a patient, a body temperature probe for measuring the body temperature of the patient, a blood pressure probe for measuring the blood pressure of the patient, an SpO₂ probe for measuring the oxygen saturation degree and pulse, and an electrocardiographic probe for measuring the electrocardiogram of the patient. The data of the sensor like a stethoscope used for consultation by hearing the sound is transmitted by changing with a microphone using a switch, and the data of the other probes are digitized by monitoring devices and are transmitted as batch data. Therefore, the sound of the stethoscope can be directly heard by using an earphone of a headset at the doctor terminal etc. and the data of other vital sensors can be displayed on a monitor screen for confirmation.

The medical examination sensor is usually worn by the patient for taking measurements, such the probes are preferably configured so as to be worn by the patient. Depending upon the condition of a disease, however, the medical examination sensor may include a highly sophisticated one that is mounted by a doctor who makes a visit. Further, the medical examination sensor may not be the same for every patient, but may be selected out of many kinds of sensors depending upon the condition of the disease.

As the video/audio communication program installed in each terminal, for example, the program corresponding to the IP-type videophone protocol in compliance with H. 323 recommended by ITU-T may be used, since the internet is used here as the communication line. When an ISDN public line is used as the communication line, the program corresponding to the ISDN videophone protocol in compliance with H. 320 recommended by ITU-T may be used. However, the video/audio communication program is not limited thereto, and any kind of program may be used as long as it is capable of bidirectional communication of video and audio through the communication line.

In the above-described embodiment, the terminals are provided with a computer having a video input interface, audio input/output interface and network communication device, in which the video/audio communication program and the web browser are installed. However, this invention is not limited thereto, and any kind of terminal may be used as long as it includes the videophone function and the web browser function.

In the above-described embodiment, the consultation room 1, consultation room 2, consultation room 3..., and individual conversation room A, individual conversation room B, individual conversation room C, individual conversation room D, individual conversation room E..., communicate with the terminals in a one-to-one manner. Therefore, the communication needs not necessarily pass through the communication server, and the terminals may be directly connected through the videophone. In this case, the reception server may be provided with a function for mediating the IP address over to the terminals to which the connection is to be made.

The above-described embodiment is provided with the communication server 120 providing a multi-point connection for connecting three or more videophone terminals, such that the waiting room terminal is mutually connected to a plurality of patient terminals in the virtual waiting room. Here, the video of the terminals that are received may be synthesized and delivered to the terminals, or the video of the terminals may be transmitted to each of the terminals and displayed being rearranged on each terminals.

Further, the nursing room 1, nursing room 2, nursing room 3... are used to confirm the video and audio of the patient terminals who receive nursing when the nurse terminal or the doctor terminal is connected, and transmit the video of the patient terminals entered in the virtual nursing room to the nurse terminal or the doctor terminal upon a request from the nurse terminal or the doctor terminal. At this moment, the video of the patient terminals may be synthesized and delivered to the nurse terminals or the doctor terminals, or the video of the patient terminals may be transmitted to the nurse terminals or the doctor terminals and displayed being rearranged on the receiving side.

Next, a procedure for performing the at-home medical consultation using the at-home medical consultation system 100 is described.

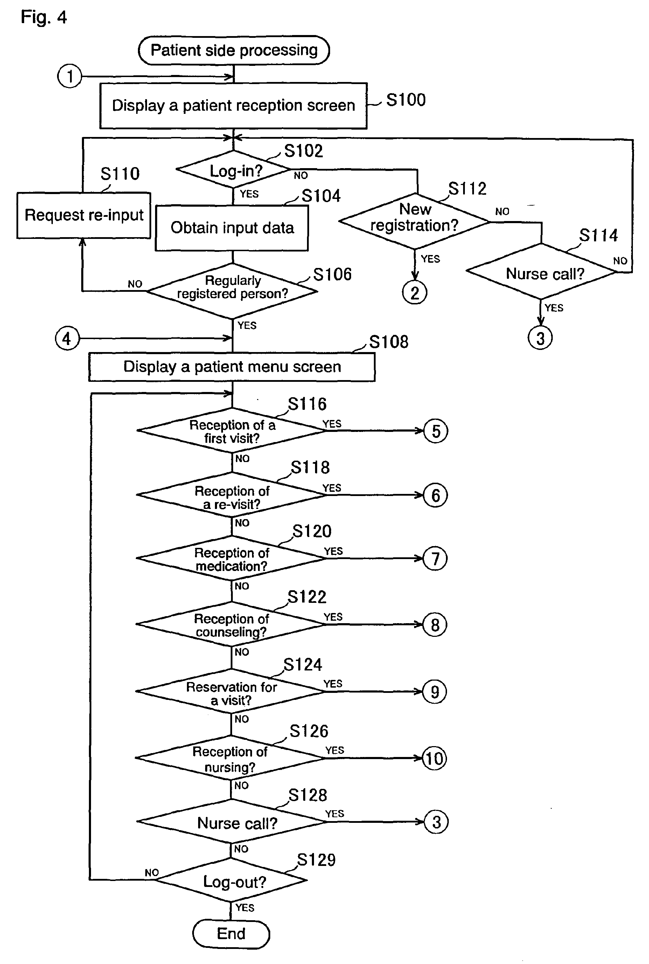

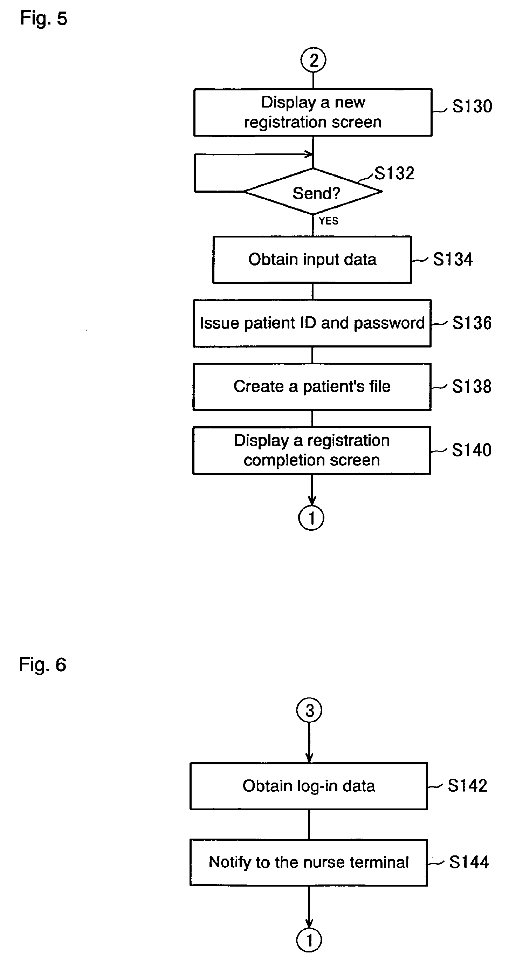

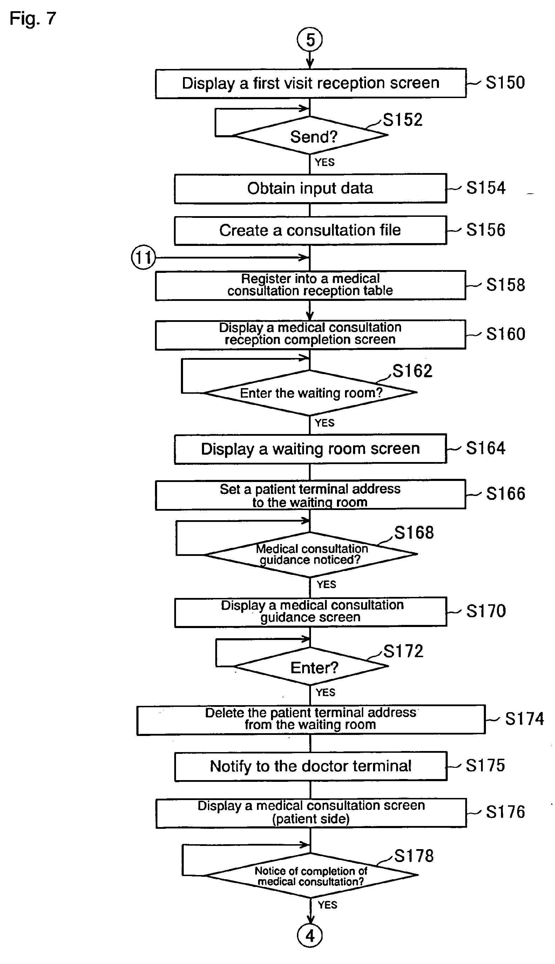

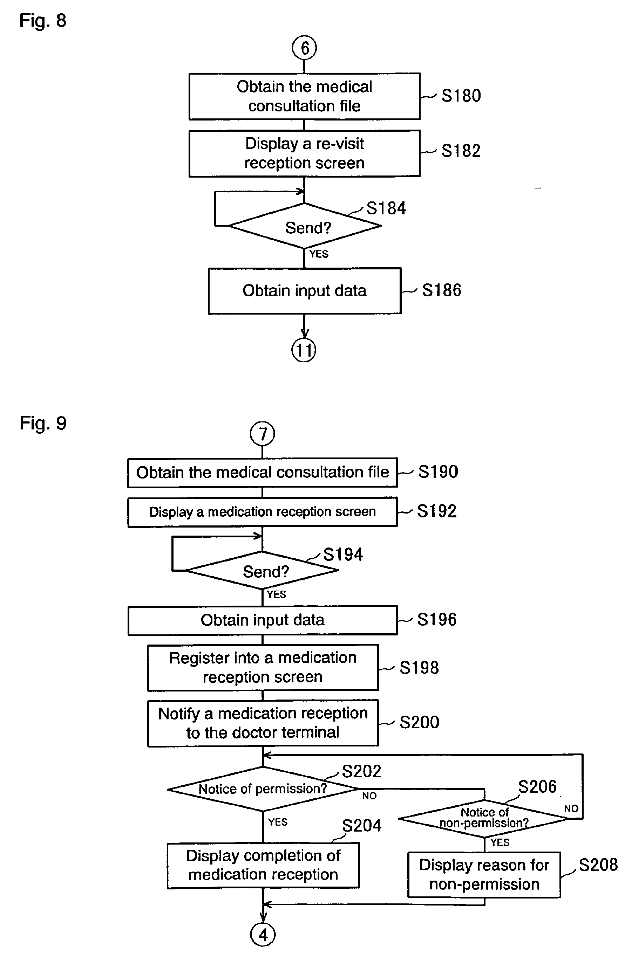

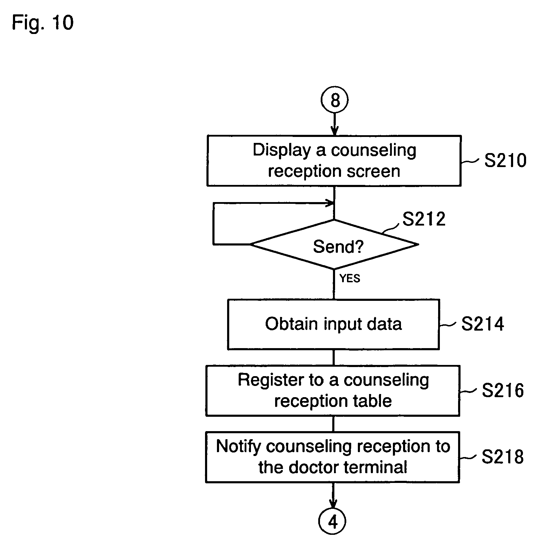

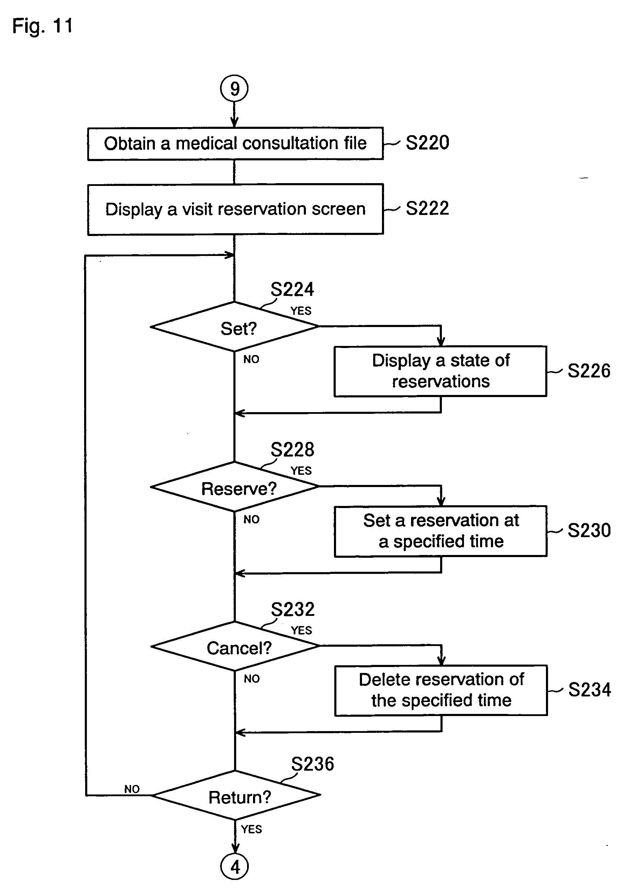

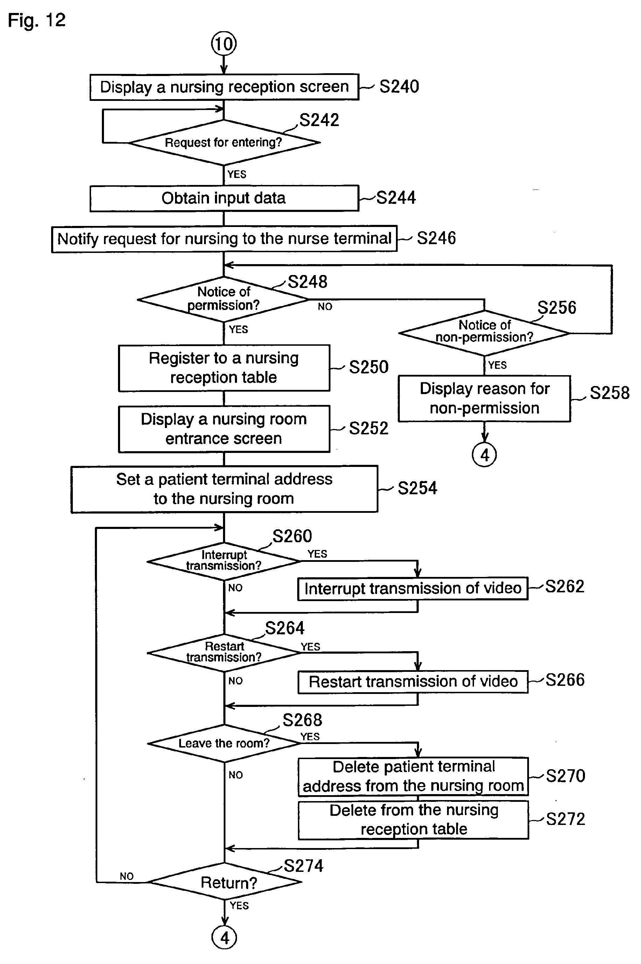

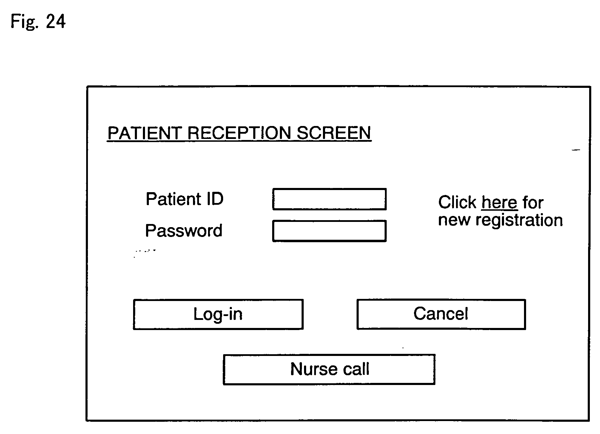

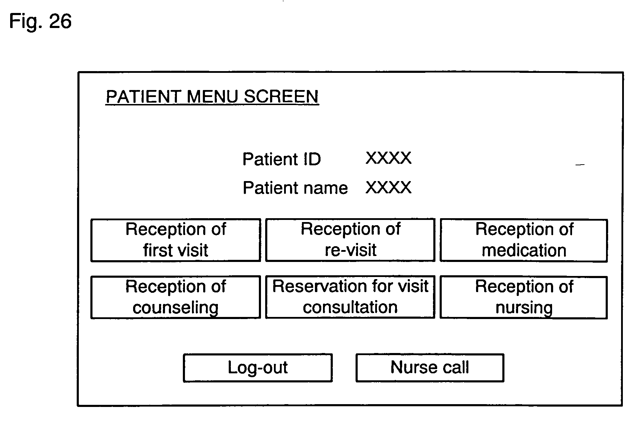

Figs. 4 to 12 illustrate flowcharts of the patient-side processing in the reception server 110. When a patient accesses a predetermined address from the patient terminal, a patient reception screen is displayed (S100) as shown in Fig. 24. When a predetermined patient ID and a password are input and a "log-in" button is clicked on the patient reception screen (S102), the input data is obtained (S104) and collated with the patient's file. When the patient is a regularly registered person (S106), a patient menu screen shown in Fig. 26 is displayed (S108). When the patient is not a regularly registered person, a screen (not shown) for requesting the re-input is displayed (S110).

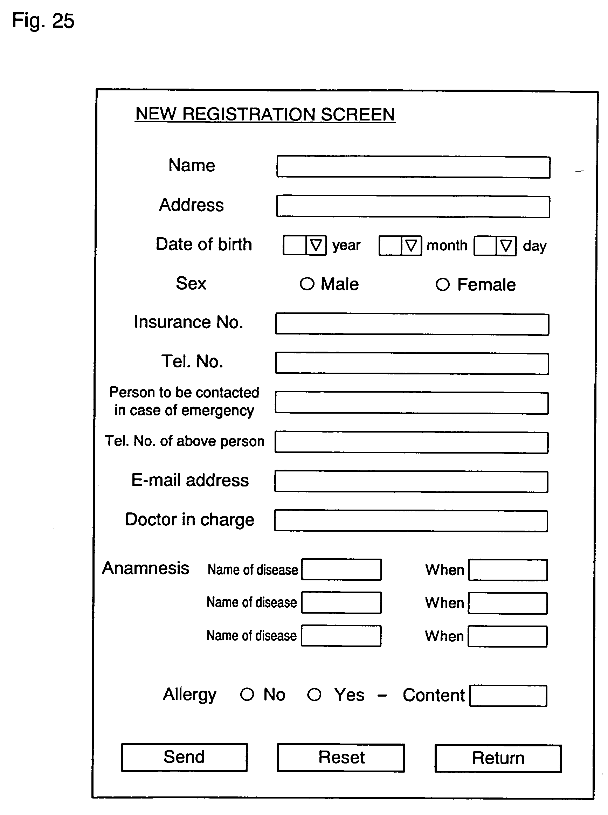

When the patient has not been registered, "HERE" is clicked to display a new registration screen as shown in Fig. 25 (S130). On the new registration screen, the name, address, date of birth, sex, insurance number, telephone number, the name of a person who should be called in case of an emergency, telephone number of the person who should be called in case of an emergency, E-mail address, name of a doctor in charge, anamnesis, allergy, etc. are input. When a "SEND" button is clicked (S132), the input data are obtained (S134), and the patient ID and password are issued (S136). Further, a patient's file is created (S138), and the input data are recorded in the patient's file together with the issued patient ID and password. Then, a registration completion screen (not shown) is displayed (S140), and the routine returns to the patient reception screen of S100.

When a "NURSE CALL" button is clicked on the patient reception screen, or when the nurse call button provided at the patient terminal is pressed (S114), the log-in data at the patient terminal are obtained (S142), a nurse call from the patient is notified to the nurse terminal (S144), and the routine returns to the patient reception screen of S100. In case of an emergency, therefore, the patient can conduct a conversation with a nurse through nurse-side processing described later without going through the log-in procedure.

After the log-in from the patient reception screen, the patient can call the nurse terminal at any time through the same processing by clicking the "NURSE CALL" button displayed on the screen or pressing the nurse call button provided at the patient terminal.

In the following procedure, the description of the nurse call is not repeated since it is the same.

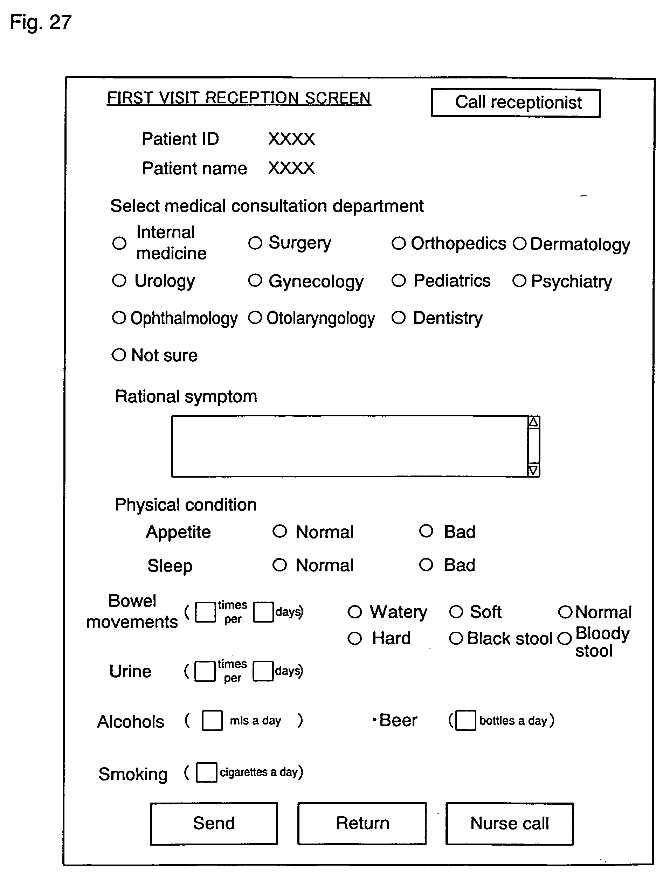

When the "FIRST VISIT RECEPTION" button is clicked on the patient menu screen (S116), a first visit reception screen shown in Fig. 27 is displayed (S150). A medical consultation department desired by the patient is selected on the first visit reception screen, and data related to the conditions of the body, such as rational symptoms, appetite, sleep, bowel movements, urine, drinking, smoking, etc. are input. When a "SEND" button is clicked (S152), the input data are obtained (S154), and a patient's consultation file on the selected consultation departments is created (S156).

The first visit reception screen is provided with a "RECEPTIONIST CALL" button. When the patient clicks the "RECEPTIONIST CALL" button, a notice of the call is transmitted to the reception terminal. When the reception terminal responds to this, a terminal address of the reception terminal and a terminal address of the patient terminal are set to an individual conversation room in the destination table 122 in the communication server, such that conversation can be held by video and audio between the patient terminal and the reception terminal in the hospital. Therefore, the patient can consult the receptionist when he has no idea of the medical consultation department or does not know how to perform the procedure.

The following reception screen also includes the "CALL RECEPTIONIST" button which, however, is not described again since it is the same.

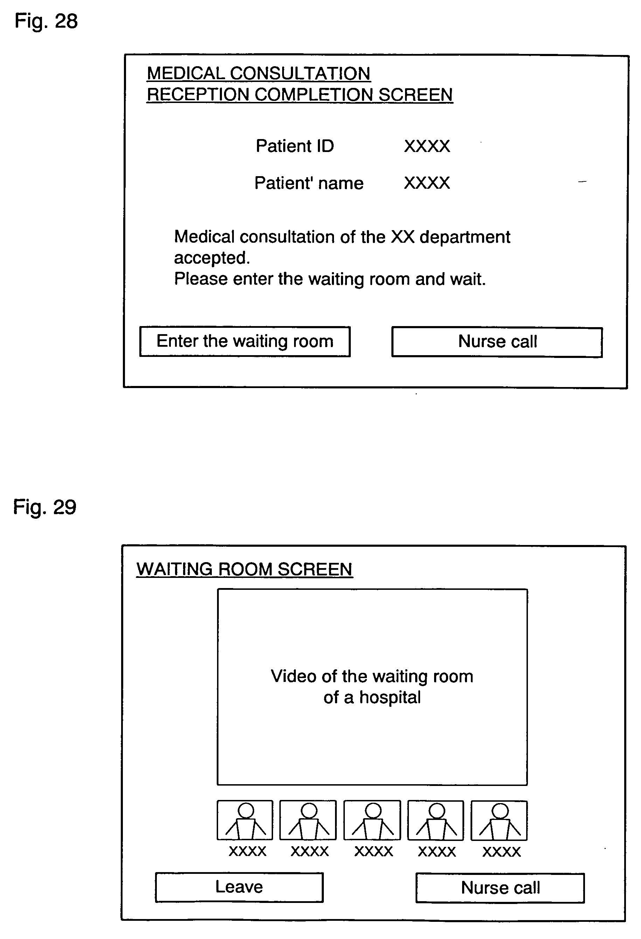

When a medical consultation is accepted, the patient is registered to the medical consultation reception table (S158) and a medical consultation reception completion screen shown in Fig. 28 is displayed (S160). When an "ENTER THE WAITING ROOM" button is clicked on the medical consultation reception completion screen (S162) , a waiting room screen shown in Fig. 29 is displayed (S164), and a patient terminal address is set to the virtual waiting room of the destination table 122 in the communication server (S166) . Therefore, conversation can be conducted by video and audio among the patient terminals entered in the virtual waiting room and the waiting room terminal of the hospital.

The waiting room screen displays the video of the waiting room of the hospital and the video of the patients entered in the virtual waiting room, as well as the names of the patients on each video display portion of the patients. Patients who do not wish to publish their names may register their nicknames in advance, such that their registered nicknames may be displayed.

When the video display portion of a desired patient is clicked on the waiting room screen, the clicked location is obtained and is compared with the coordinates of each of the patient terminals that are displayed to select a callee patient terminal, and a notice of call (not shown) is transmitted to the patient terminal. When the callee patient clicks the "RESPONSE" button in response to the notice of call, an individual conversation screen (not shown) is displayed, and a terminal address of the caller patient terminal and a terminal address of the callee patient terminal are set to the individual conversation room A in the destination table 122 in the communication server. Thus, the patients entered in the virtual waiting room are allowed to conduct a conversation individually among themselves.

The waiting room screen which is the same as that of Fig. 29 is also displayed on the waiting room terminal 175 of the hospital. When a patient in the waiting room of the hospital clicks the video display portion of a desired patient, a notice of call (not shown) is transmitted to a caller patient terminal in the same manner as described above. When the callee patient clicks the "RESPONSE" button in response to the notice of call, an individual conversation screen (not shown) is displayed, and a terminal address of the waiting room terminal and a terminal address of the callee patient terminal are set-to the individual conversation room in the destination table 122 in the communication server. Thus, the patient in the waiting room of the hospital is allowed to conduct a conversation individually with the patient entered in the virtual waiting room.

When a "LEAVE" button is clicked on the waiting room screen, the terminal address of the patient terminal is deleted from the waiting room of the destination table 122 in the communication server, and the connection to the virtual waiting room is terminated.

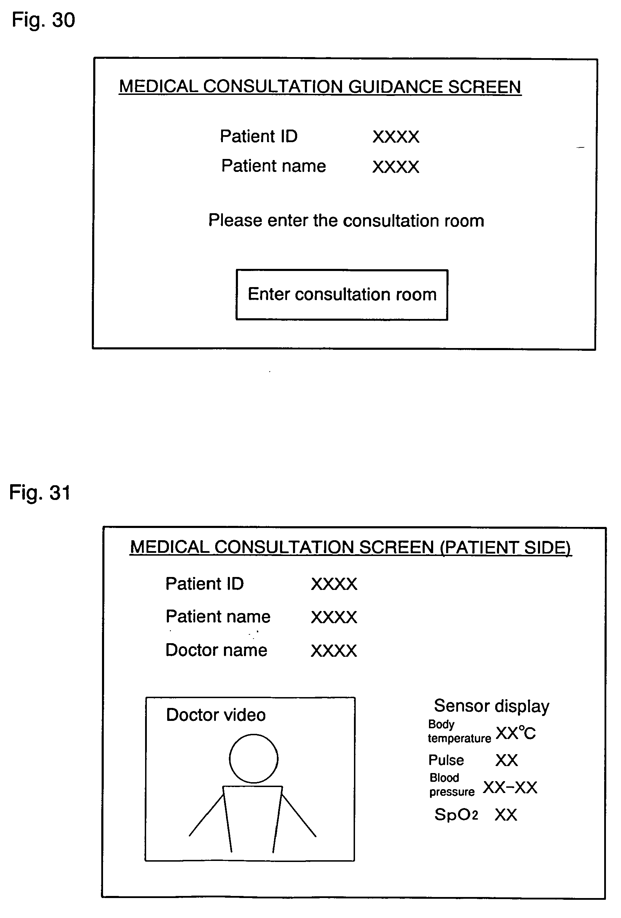

Upon receipt of a notice of medical consultation guidance from the nurse terminal or the doctor terminal (S168) , a medical consultation guidance screen is displayed as shown in Fig. 30 (S170). When an "ENTER CONSULTATION ROOM" button is clicked in the medical consultation guidance screen (S172), the terminal address of the patient terminal is deleted from the waiting room of the destination table 122 in case the patient has entered in the virtual waiting room (S174) , the doctor in charge's terminal (S175) is notified of the entry into the consultation room, and the medical consultation screen (patient side) shown in Fig. 31 is displayed (S176). The patient terminal is connected to the doctor in charge's terminal through the doctor side processing described below, and the patient is allowed to conduct a conversation with the doctor in charge and receive a medical consultation from the doctor.

In conducting the medical consultation, the patient wears the medical examination sensors such that the doctor terminal can take vital measurements and conduct the medical examination through a stethoscope in a remote operation. Values measured by the medical examination sensors are displayed on the consultation screen so that the patient can also see the measured values.

Upon receipt of a notice of completion of medical consultation from the doctor terminal (S178), the medical consultation screen (patient side) is closed and the routine returns to the patient menu screen of S108.

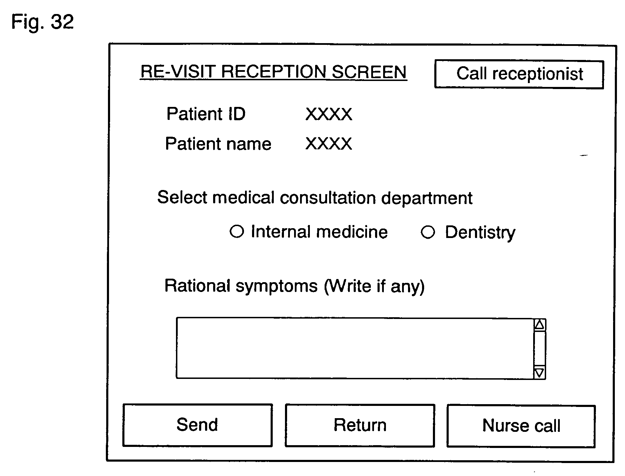

When a "RE-VISIT RECEPTION" button is clicked on the patient menu screen (S118), the medical consultation file of the patient is obtained (S180) and a re-visit reception screen is displayed as shown in Fig. 32 (S182). The medical consultation department that is now being held is displayed on the re-visit reception screen based on the medical consultation file. When a desired medical consultation department is selected out of the departments that are displayed on the re-visit reception screen, a rational symptom is input, if any, and a "SEND" button is clicked (S184), the input data are obtained (S186) and the routine proceeds to S158. Thus, the patient is registered to the medical consultation reception table as in the case of accepting the first visit described above, and waits for the notice of medical consultation guidance to consult the doctor.

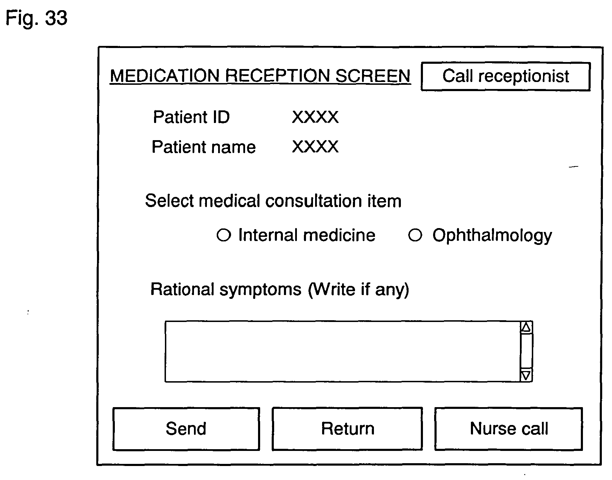

When an "MEDICATION RECEPTION" button is clicked on the patient menu screen (S120), the medical consultation file of the patient is obtained (S190) and a medication reception screen as shown in Fig. 33 is displayed (S192). The medical consultation department that is now being held is displayed on the medication reception screen based on the medical consultation file. When a desired department of medication is selected out of the departments that are displayed on the medication reception screen, a rational symptom is input, if any, and a "SEND" button is clicked (S194), the input data are obtained (S196), and are registered in an medication reception table (S198), the doctor terminal is notified of the medication reception (S200). When a notice of permission is received from the doctor terminal in response to the notice of medication reception through the doctor side processing described below (S202), a completion of medication reception (not shown) is displayed (S204). When a notice of non-permission is received (S206), the reason for non-permission (not shown) is displayed (S208) and the routine returns to the patient menu screen of S108.

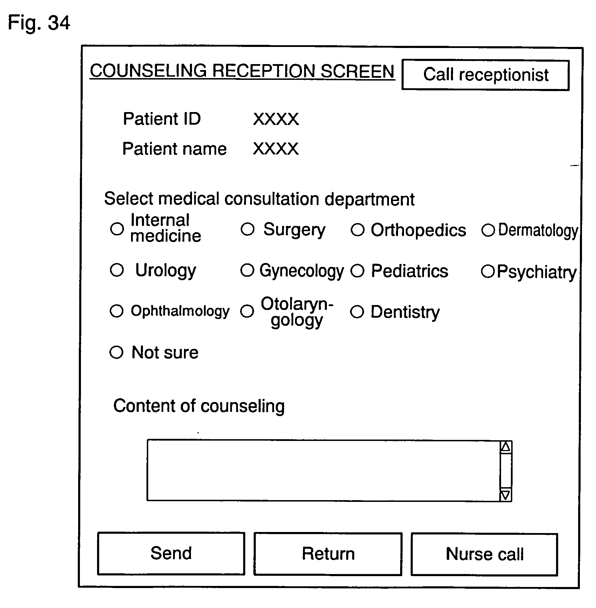

When a "COUNSELING RECEPTION" button is clicked on the patient menu screen (S122), a counseling reception screen as shown in Fig. 34 is displayed (S210). When a desired department of counseling is selected on the counseling reception screen, the content of counseling is input, and a "SEND" button is clicked (S212), the input data are obtained (S214), a notice of counseling reception is transmitted to the doctor terminal (S218), and the routine returns to the patient menu screen at S108. Thus, a reply of the doctor in charge is transmitted by mail to the patient terminal at a later date through the doctor side processing described below.

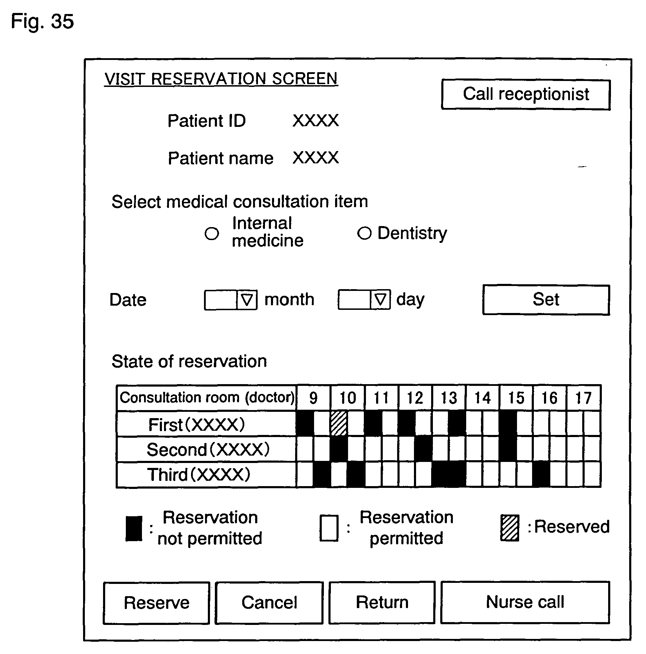

When a "VISIT RESERVATION" button is clicked on the patient menu screen (S124), the medical consultation file of the patient is obtained (S220) and a visit reservation screen as shown in Fig. 35 is displayed (S222). The departments of medical consultation that is now being held are displayed on the visiting reservation screen based on the medical consultation file. When a desired department of visit consultation is selected out of the departments that are displayed, a date is input, and the "SET" button is clicked (S224) so as to display a state of reservations (S226). When the possible reservation time that can be reserved is selected in the state of reservation display, and a "RESERVE" button is clicked (S228), the specified time is displayed as being reserved, and the reservation of the specified time is set to the visit reservation table (S230). Thus, the patient is allowed to make a reservation of visit from the patient terminal.

When the specified time that has been reserved is selected and a "CANCEL" button is clicked (S232), the display of reservation of the specified time is deleted, and the reservation is deleted from the visit reservation table (S234) . Thus, the patient is allowed to cancel the reservation of visit from the patient terminal.

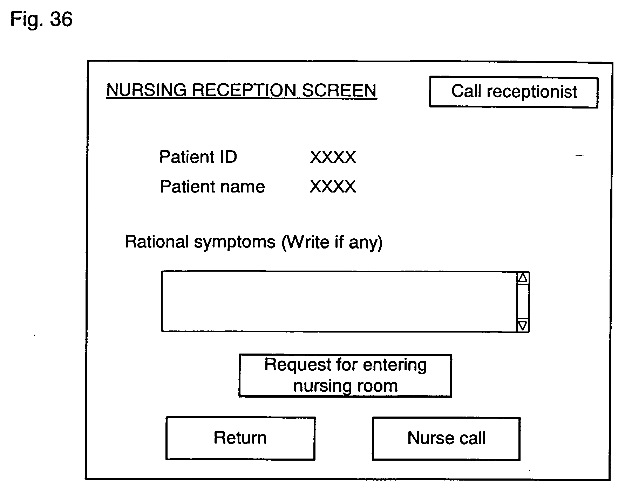

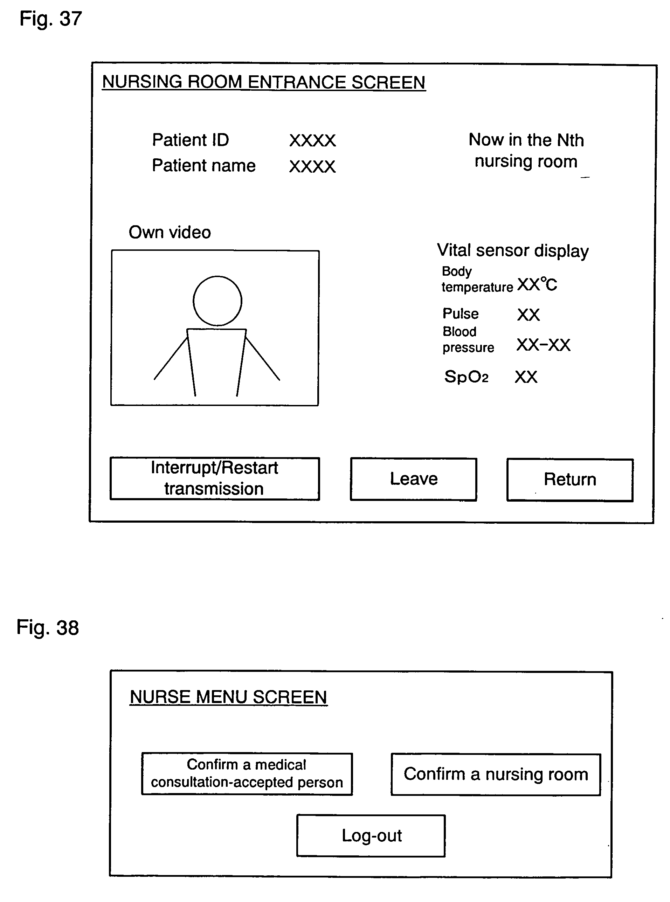

When a "NURSING RECEPTION" button is clicked on the patient menu screen (S126), a nursing reception screen is displayed as shown in Fig. 36 (S240). When a rational symptom is input, if any, and a "REQUEST FOR ENTERING NURSING ROOM" button is clicked on the nursing reception screen (S242), the input data are obtained (S244) and the nurse terminal is notified of a request for nursing (S246). When a notice of permission is received from the nurse terminal in response to the request for nursing through the nurse side processing described below (S248), the patient is registered to the nursing reception table (S250), a nursing room entrance screen shown in Fig. 37 is displayed (S252), and a terminal address of the patient terminal is set to the nursing room in the destination table 122 (S254). When a notice of non-permission is received from the nurse terminal (S256), the reason for non-permission is displayed (S258) and the routine returns to the patient menu screen of S108.

When an "INTERRUPT TRANSMISSION" button is clicked on the nursing room entrance screen (S260), the transmission of video and audio is interrupted (S262) and when a "RESTART TRANSMISSION" button is clicked (S264), the transmission of video and audio is restarted (S266). Thus, the patient in the virtual nursing room is allowed to keep his privacy by interrupting the transmission if he does not wish to be seen by the nurse or the doctor.

When a "LEAVE" button is clicked on the nursing room screen (S268), the terminal address of the patient terminal is deleted from the nursing room in the destination table 122 (S270), and the patient is deleted from the nursing reception table (S272). When a "RETURN" button is clicked (S274), the routine returns to the patient menu screen of S108.

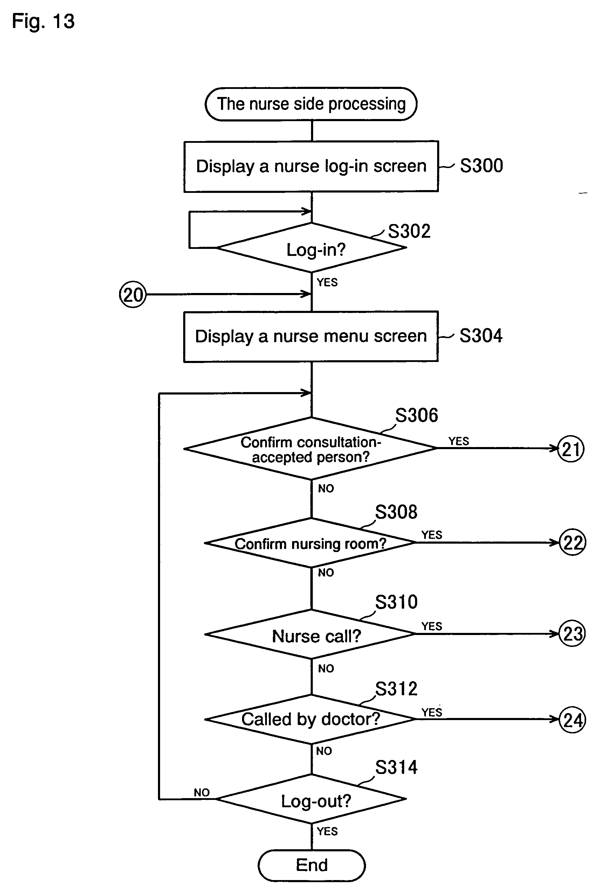

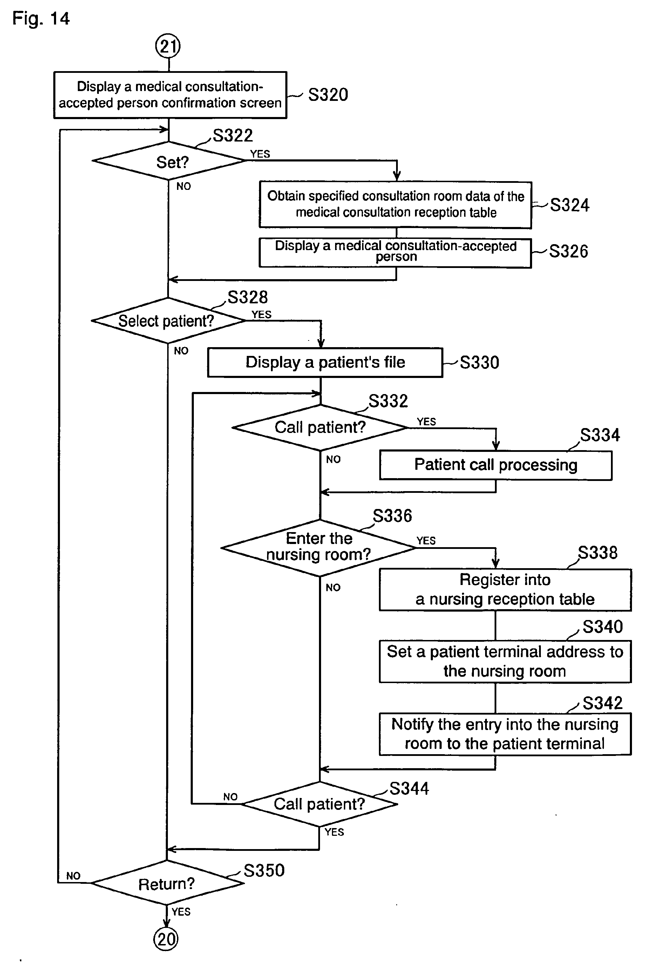

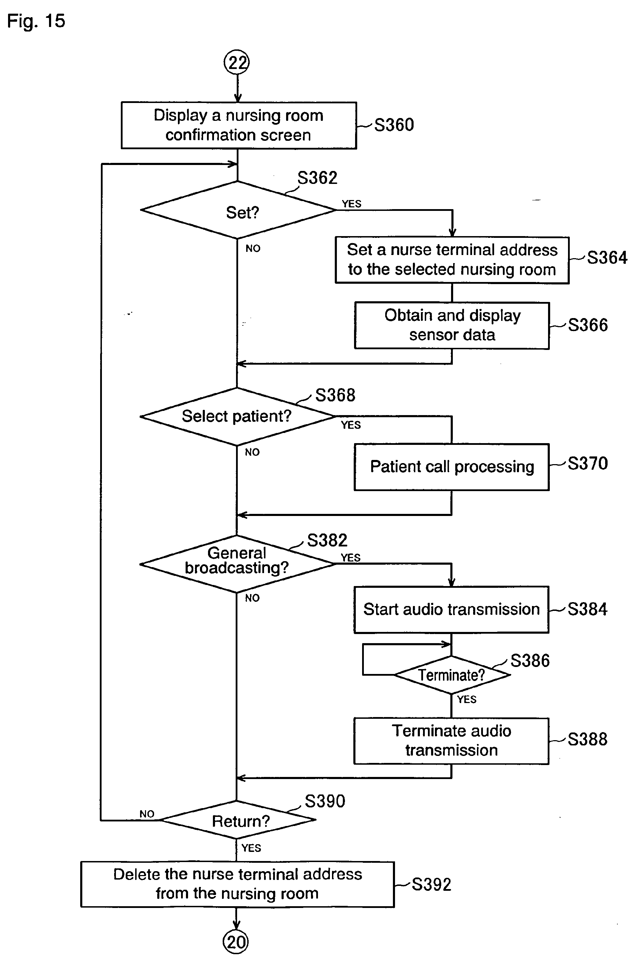

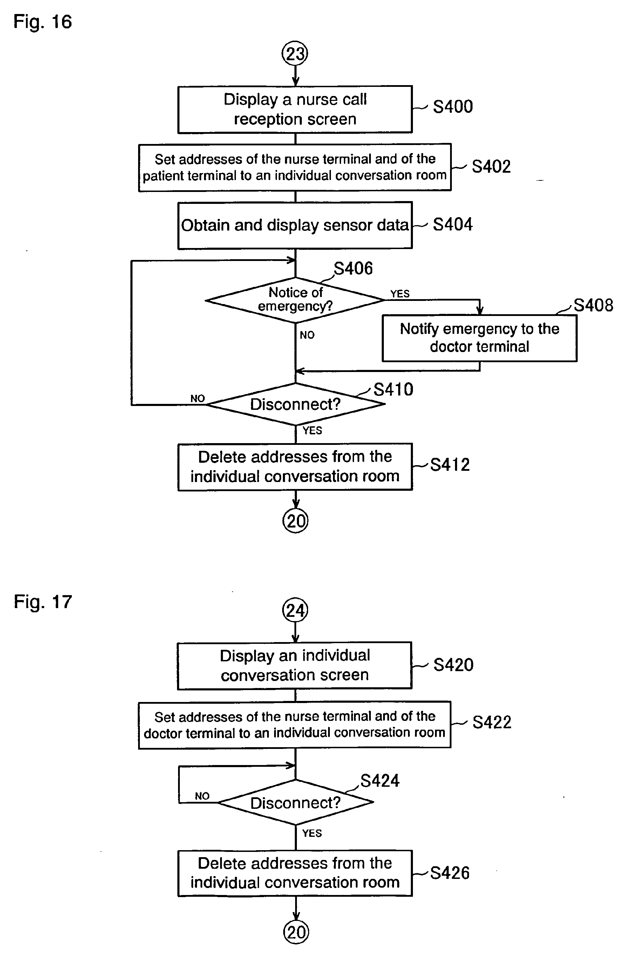

Figs. 13 to 17 illustrate flowcharts of the nurse side processing in the reception server 110. When a nurse accesses a predetermined address from the nurse terminal, a nurse log-in screen (not shown) is displayed (S300). When a predetermined nurse ID and a password are input and a "LOG-IN" button is clicked on the nurse log-in screen (S302), a nurse menu screen shown in Fig. 38 is displayed (S304).

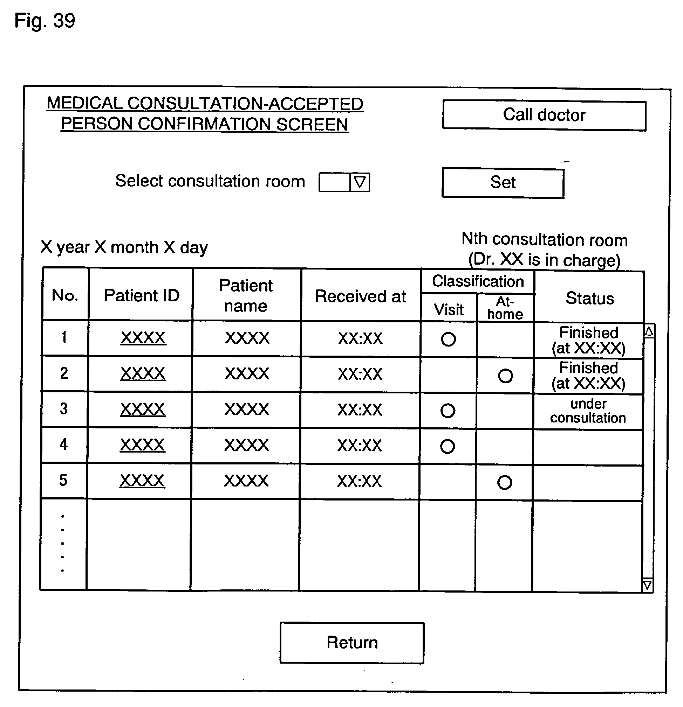

When a "CONFIRM MEDICAL CONSULTATION ACCEPTED PERSON" button is clicked on the nurse menu screen (S306), a medical consultation accepted person confirmation screen shown in Fig. 39 is displayed (S320).

When a consultation room is selected and a "SET" button is clicked on the medical consultation accepted person confirmation screen (S322), the data of the specified consultation room of the medical consultation reception table is obtained(S324), and a medical consultation accepted persons' list of the day is displayed (S326).

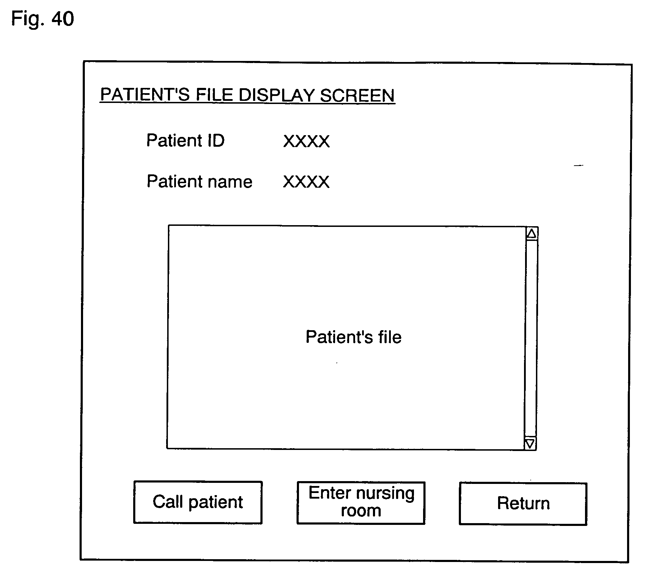

When the patient ID of a particular patient is clicked in the medical consultation accepted persons' list (S328), a patient's file display screen shown in Fig. 40 is displayed (S330) such that the patient's file data can be confirmed.

The patient's file display screen is provided with a "CALL PATIENT" button. When this button is clicked (S332), a processing for calling the patient is executed (S334). Then a notice of call to the patient terminal is transmitted, an individual conversation screen is displayed and a terminal address of the patient terminal and a terminal address of the nurse terminal are set to the individual conversation room in the destination table such as the nurse call reception described below, such that a conversation by video and audio between the patient and the nurse can be conducted. In case of an emergency, an "EMERGENCY NOTICE" button is clicked to notify the doctor terminal.

Further, the patient's file displaying system is provided with an "ENTER NURSING ROOM" button. When this button is clicked (S336), the patient is registered to the nursing reception table (S338), an address of the patient terminal is set to the nursing room in the destination table 122 (S340), the patient terminal is notified of the entry into the nursing room (S342). On the other hand, when a "DISPLAY OF NURSING ROOM ENTRANCE" button provided in the notice of entry into the nursing room is clicked at the patient terminal, the above-mentioned nursing room entrance screen is displayed.

When a "RETURN" button is clicked on the patient's file displaying screen (S344), the routine returns back to the medical consultation accepted person confirmation screen. When a "RETURN" button is clicked on the medical consultation accepted person confirmation screen (S350), the routine returns to the nurse menu screen of S304.

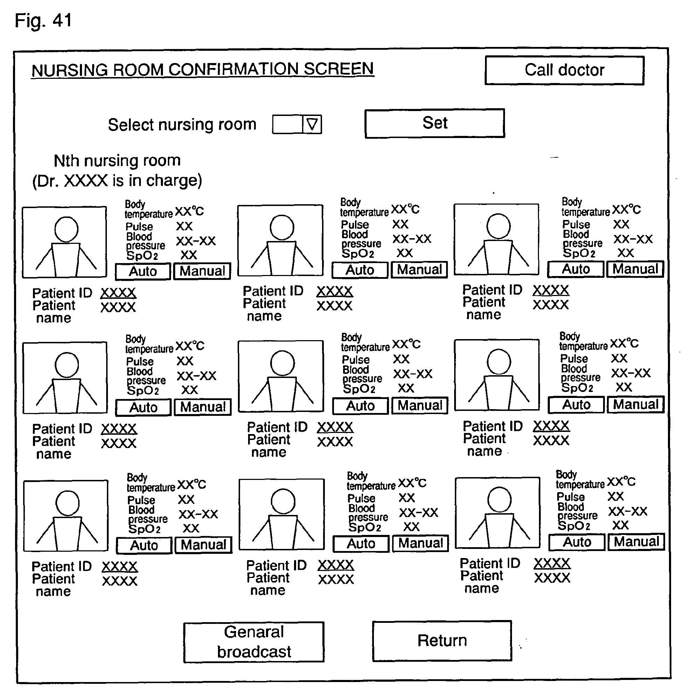

When a "CONFIRM NURSING ROOM" button is clicked on the nurse menu screen (S308), a nursing room confirmation screen as shown in Fig. 41 (S360) is displayed. When a nursing room is selected, and a "SET" button is clicked (S362), the address of the nurse terminal is set to the selected nursing room in the destination table 122 (S364) to obtain and display the video and audio of the patient terminals in the nursing room, as well as to obtain and display the medical examination sensor data at the patient terminals (S366).

The sensor display portion of every patient is provided with an "AUTOMATIC" button and a "MANUAL" button. When the "AUTOMATIC" button is clicked, the medical examination sensor takes a measurement at regular intervals. When the "MANUAL" button is clicked, the automatic measurement is reset and the measurement is taken on demand.

When the video of a particular patient is clicked on the nursing room confirmation screen, the clicked position is obtained and is compared with the coordinates of each patient terminal that are displayed to select the callee patient terminal (S368), thereby to execute processing for calling the patient (S370). Thus, an individual conversation screen (not shown) is displayed, a terminal address of the patient terminal and a terminal address of the nurse terminal are set to the individual conversation room in the destination table, such that a conversation can be conducted by video and audio between the patient and the nurse, such as the nurse call reception described below. When an "EMERGENCY NOTICE" button is clicked on the individual conversation screen, the doctor in charge's terminal is notified of such, so that the doctor can deal with a sudden deterioration in the patient's condition.

The nursing room confirmation screen is provided with a "GENERAL BROADCAST" button. When this button is clicked (S382), the audio is transmitted to the patient terminals (S384). When the click is reset (S386), transmission of the audio to the patient terminals terminates (S388). Thus, all of the patients entered in the nursing rooms can be informed at one time. Here, although the general broadcast is by audio only, the video of the nurse terminal may be transmitted to, and displayed on, the patient terminals.

When a "RETURN" button is clicked on the nursing room confirmation screen (S392), the address of the nurse terminal is deleted from the nursing room in the destination table 122 (S392) and the routine returns to the nurse menu screen at S304 .

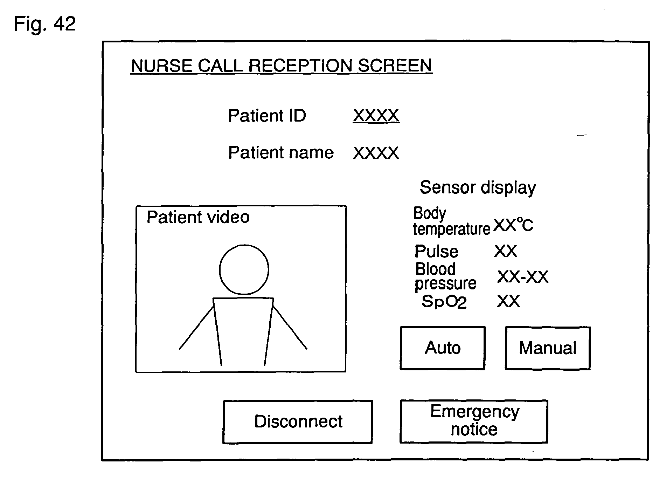

When a notice of nurse call is received from the patient terminal on the nurse menu screen (S310) , a nurse call reception screen shown in Fig. 42 is displayed (S400), the terminal address of the nurse terminal and the terminal address of the patient terminal are set to the individual conversation room in the destination table 122 (S402), and the medical examination sensor data at the patient terminal are obtained and displayed (S404). Therefore, the nurse can conduct a conversation with the patient by video and audio, and confirm the medical examination sensor data of the patient so that a suitable treatment for the patient is performed.

When an "EMERGENCY NOTICE" button is clicked on the nurse call screen (S406) , the doctor in charge's terminal is notified of an emergency (S408). When a "CONFIRM PATIENT" button provided in the emergency notice is clicked at the doctor in charge's terminal, the patient terminal is connected to the doctor terminal through the doctor side processing described below, such that the doctor in charge is allowed to confirm the video and audio of the patient as well as the medical examination sensor data.

When a "DISCONNECT" button is clicked on the nurse call reception screen (S410), the terminal addresses of the nurse terminal and of the patient terminal are deleted from the individual conversation room in the destination table 122 (S412), and the routine returns to the nurse menu screen of S304.

When a notice of call is received from the doctor on the nurse menu screen (S312), an individual conversation screen (not shown) is displayed (S420), and the terminal address of the nurse terminal and the terminal address of the doctor terminal are set to the individual conversation room in the destination table 122 (S422) . Thus, a conversation can be held by video and audio between the nurse and the doctor. When a "DISCONNECT" button is clicked on the individual conversation screen (S424), the terminal addresses of the nurse terminal and the doctor terminal are deleted from the individual conversation room in the destination table 122 (S426), and the routine returns to the nurse menu screen at S304.

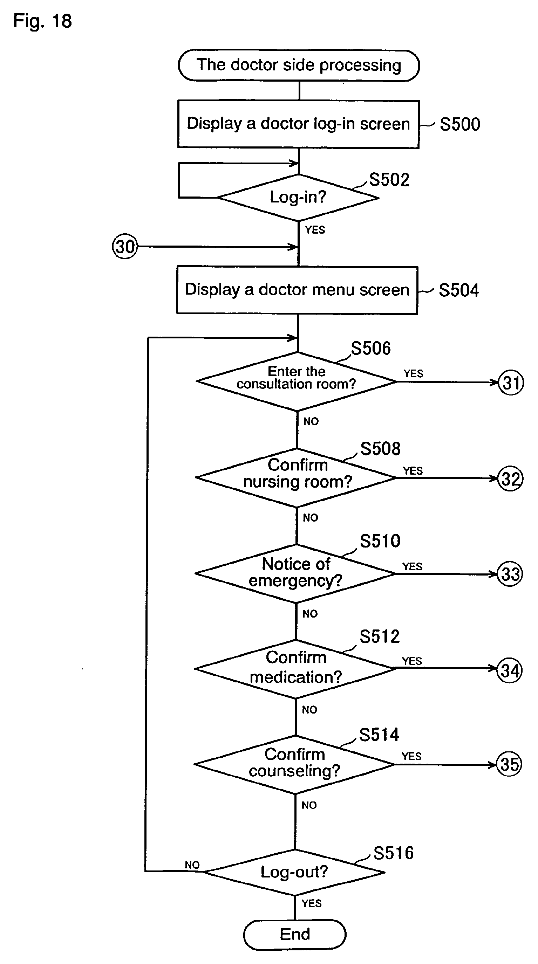

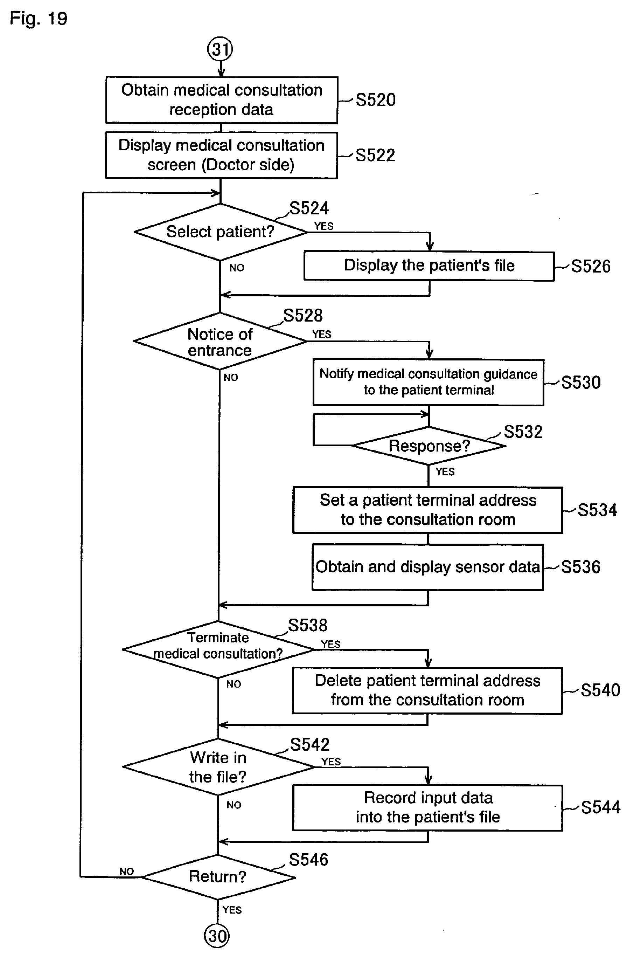

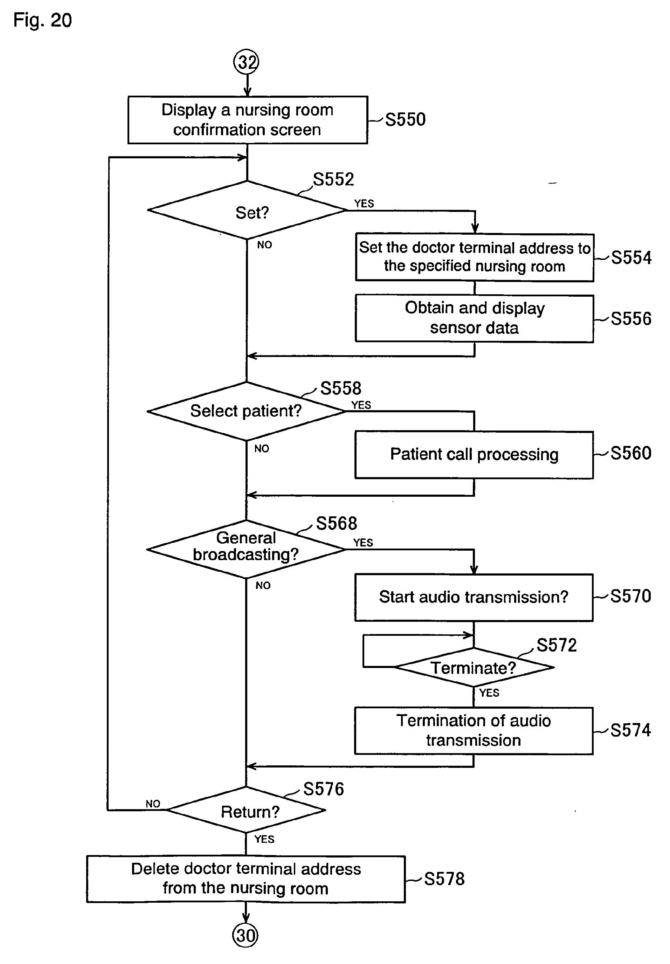

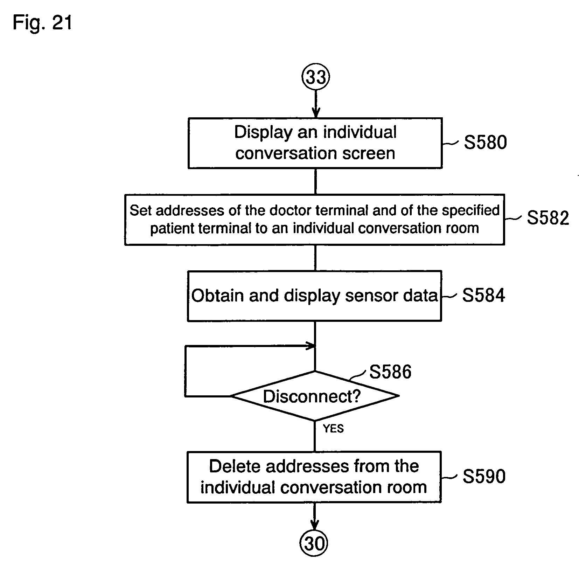

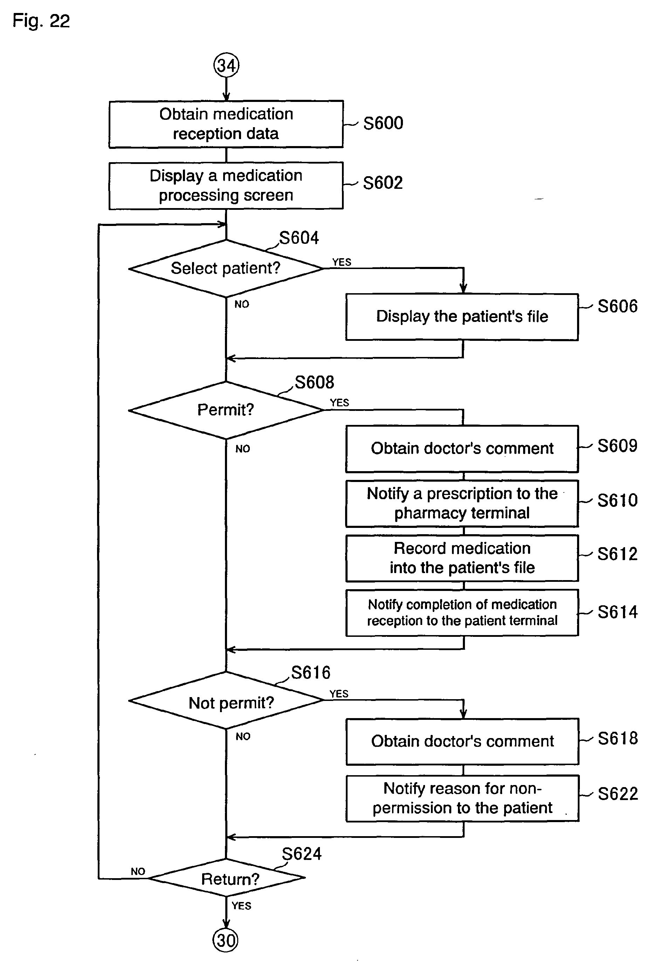

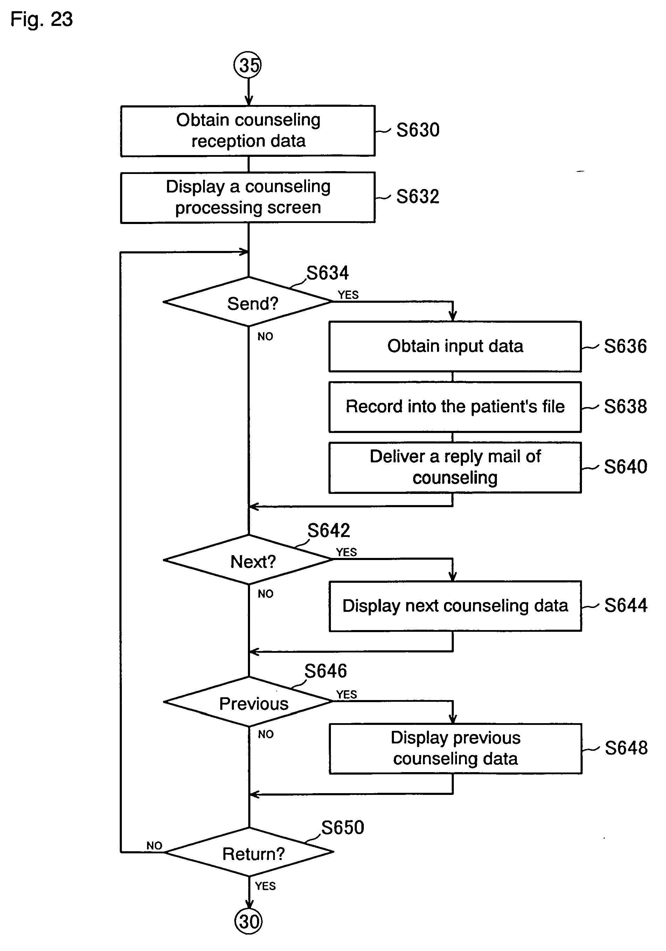

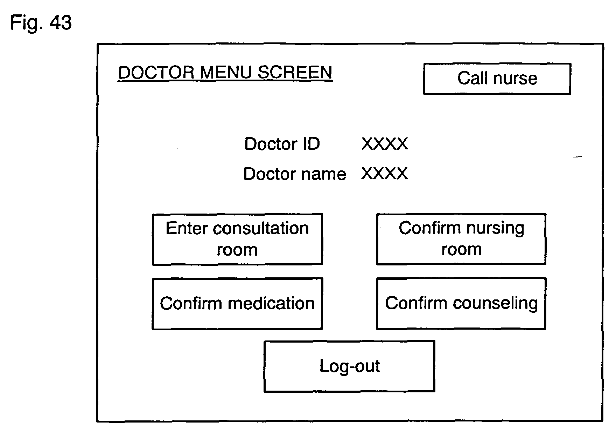

Figs. 18 to 23 illustrate flowcharts of a doctor side processing in the reception server 110. When the doctor accesses a predetermined address from the doctor terminal, a doctor log-in screen (not shown) is displayed (S500). When a predetermined doctor ID and a password are input and a "LOG-IN" button is clicked on the doctor log-in screen (S502), a doctor menu screen shown in Fig. 43 is displayed (S504).

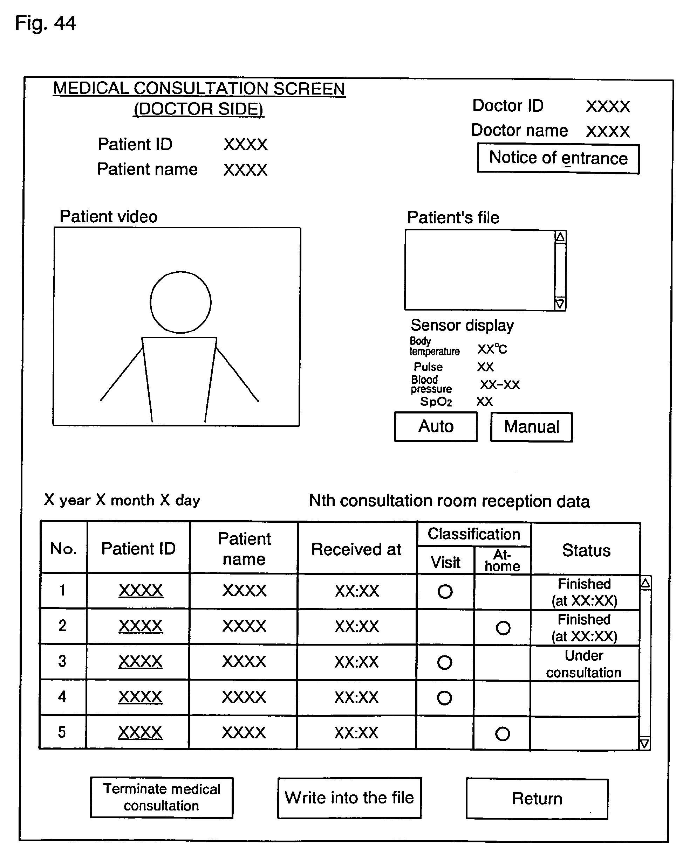

When an "ENTER THE CONSULTATION ROOM" is clicked on the doctor menu screen (S506), the medical consultation reception data of the consultation room in charge of the doctor from the medical consultation reception table can be obtained (S520), and a medical consultation screen (doctor side) shown in Fig. 44 is displayed (S520). The medical consultation screen (doctor side) includes a patient video display portion for displaying the video of the patient during the medical consultation, a sensor data display portion for displaying the medical examination sensor data at the patient terminal during the medical consultation, a patient's file display portion for displaying a selected patient's file, and a medical consultation reception data display portion for displaying the medical consultation reception data in the consultation room used by the doctor.

The patients who wish to receive a consultation on the day including outpatients and at-home patients in order of reception are displayed in the medical consultation reception data display portion. The patients who have already been consulted are displayed as "FINISHED" and the patients during consultation are displayed as "UNDER CONSULTATION" on the status column.

When the ID of a particular patient is clicked to select the patient in the medical consultation reception data display portion (S524), the file of the patient is retrieved and is displayed on the patient's file display portion (S526).

When a "NOTICE OF ENTRANCE" button on the right upper side on the medical consultation screen (doctor side) is clicked (S528), a notice of medical consultation guide is transmitted to the patient terminal that the patient's file is displayed (S530). When a "RESPONSE" button is clicked at the patient terminal that has received the notice (S532), the terminal address of the patient terminal is set to the consultation room in the destination table 122 (S534), and the medical examination sensor data at the patient terminal are obtained and displayed (S536).

When a "TERMINATE MEDICAL CONSULTATION" button is clicked on the medical consultation screen (doctor side), while a particular patient is receiving a medical consultation (S538) , the terminal address of the patient terminal is deleted from the consultation room in the destination table 122 (S540), and the medical consultation of the patient is completed.

When a "WRITE IN THE FILE" button is clicked on the medical consultation screen (doctor side) (S542), the data input by the doctor are obtained and are recorded into the patient's file that is open (S544).

When a "RETURN" button is clicked on the medical consultation screen (doctor side) (S546), the routine returns back to the doctor menu screen of S504.

When a "CONFIRM THE NURSING ROOM" button is clicked on the doctor menu screen (S508), a nursing room confirmation screen same as Fig. 41 is displayed (S550). When a nursing room is selected and a "SET" button is clicked (S552), a terminal address of the doctor terminal is set to the specified nursing room in the destination table 122 (S554), the video and audio of the patient terminals entered in the nursing room are obtained and displayed, and the medical examination sensor data is also displayed at the patient terminals (S556).

The sensor display portion of every patient is provided with an "AUTOMATIC" button and a "MANUAL" button. When the "AUTOMATIC" button is clicked, the consultation sensor takes a measurement at regular intervals. When the "MANUAL" button is clicked, the automatic measurement is reset, and a measurement is taken on demand.

When the video of a particular patient is clicked on the nursing room confirmation screen, the clicked position is obtained and is compared with the coordinates of the patient terminals that are displayed to select a patient callee terminal (S558) and to execute a processing for calling the patient (S560). Thus, an individual conversation screen is displayed, and the terminal address of the patient terminal and the terminal address of the terminal are set to the individual conversation room in the destination table, similar to the above-described processing on the nurse side, such that a conversation can be conducted by video and audio between the patient and the doctor.

The nursing room confirmation screen is provided with a "GENERAL BROADCAST" button. When this button is clicked (S568), the audio is transmitted to the patient terminals (S570). When the click is reset (S572), transmission of audio to the patient terminals terminates (S574). Thus, all of the patients in the nursing rooms can be informed at one time. Though the general broadcast here is by audio only, the video of the doctor terminal may be transmitted to, and displayed on, the patient terminals.

When a "RETURN" button is clicked on the screen for confirming the nursing room confirmation screen (S576), the address of the doctor terminal is deleted from the nursing room in the destination table 122 (S578) and the routine returns to the doctor menu screen of S504.

When a notice of emergency is received from the nurse terminal on the doctor menu screen (S510), an individual conversation screen (not shown) is displayed (S580), and the terminal address of the doctor terminal and the terminal address of the notified patient terminal are set to the individual conversation room in the destination table 122 (S582), and the medical examination sensor data at the patient terminal are obtained and displayed (S584). Thus, the doctor in charge can conduct a conversation with the patient by video and audio and confirm the medical examination sensor data of the patient such that a suitable treatment for the patient can be performed.

When a "DISCONNECT" button is clicked on the individual conversation screen (S586), the terminal addresses of the doctor terminal and of the patient terminal are deleted from the individual conversation room in the destination table 122 (S590), and the routine returns to the doctor menu screen of S504.

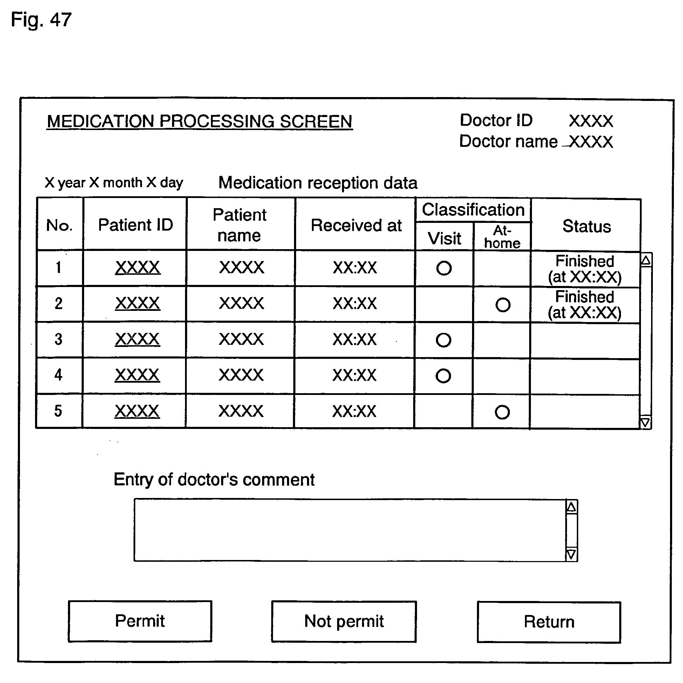

When a "CONFIRM MEDICATION" button is clicked on the doctor menu screen (S512), the medication reception data of the doctor in charge are obtained from the medication reception table (S600), and an medication processing screen shown in Fig. 47 is displayed (S602). When the ID of a particular patient is clicked to select the patient on the medication processing screen (S604), the file of the patient is obtained and is displayed (S606). When the content of medication for the patient is confirmed by the doctor in charge and a "PERMIT" button is clicked (S608), the comment input by the doctor is obtained (S609), and a prescription is sent to the pharmacy terminal (S610). Further, the medication data are recorded into the patient's file (S612) and the notice of permission of medication is transmitted to the patient terminal (S614). When a "NOT-PERMIT" button is clicked by the doctor in charge (S616), the comment input by the doctor is obtained (S618), and the reason for non-permission is transmitted to the patient terminal (S622).

When a "RETURN" button is clicked on the medication processing screen (S624), the routine returns to the doctor menu screen of S504.

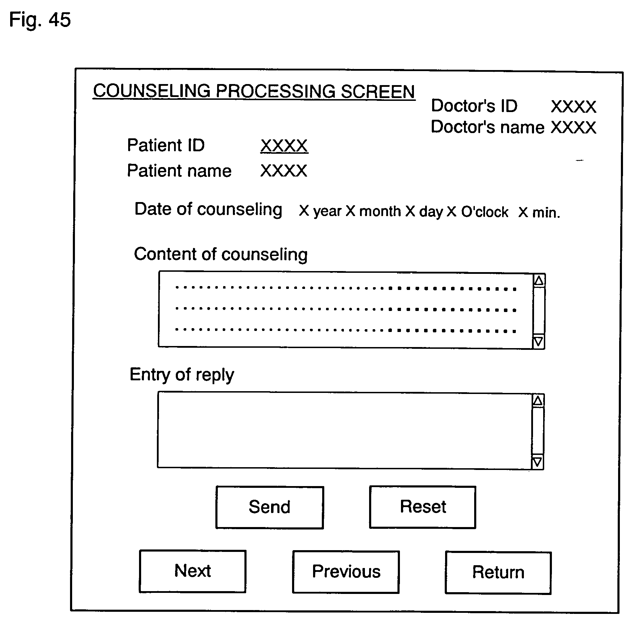

When a "CONFIRM THE COUNSELING" button is clicked on the doctor menu screen (S514), the doctor in charge's counseling reception data from the counseling reception table is obtained (S630), and a counseling processing screen shown in Fig. 45 is displayed (S632). The counseling processing screen displays the patient's ID, the patient's name, the date of counseling and the content of counseling. The doctor in charge reads the content of counseling, clicks the patient's ID, if necessary, and replies to the counseling while making reference to the patient's file.

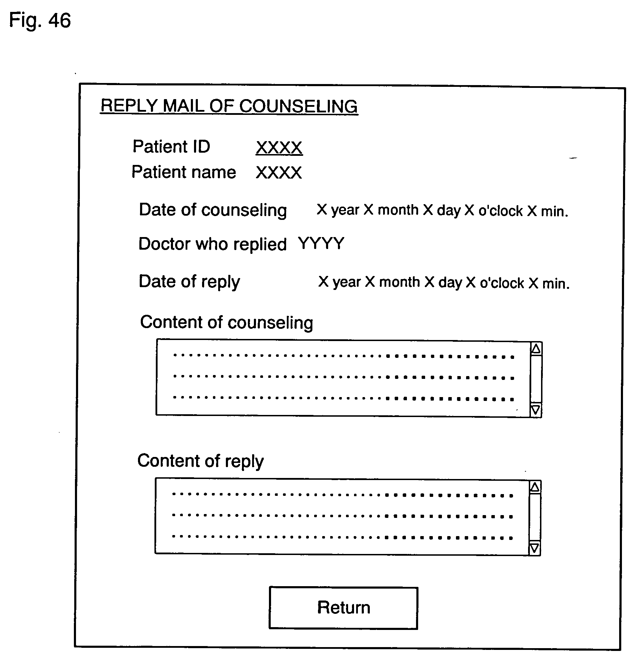

When a "SEND" button is clicked on the counseling processing screen (S634), the reply that is input is obtained (S636) and recorded in the patient's file (S638), and a reply mail of the counseling shown in Fig. 46 is generated and is delivered to the patient (S640).

When a "NEXT" button is clicked on the counseling processing screen (S642), the data of a next patient whose counseling is accepted are obtained and displayed (S644), and when a "Previous" button is clicked (S646), the data of a previous patient whose consultation was accepted are obtained and displayed (S648). Thus, the doctor in charge can confirm all registered contents of counseling and reply thereto.

When a "RETURN" button is clicked on the consultation processing screen (S650), the routine returns to the doctor menu screen of S504.

A "NURSE CALL" button is provided on the doctor menu screen, although it is omitted in the process flow in the processing on the doctor side. When this button is clicked, an individual conversation screen is displayed, and the terminal address of the doctor terminal and the terminal address of the nurse terminal are set to the individual conversation room in the destination table 122, such that a conversation can be conducted between the doctor and the nurse.

In the above-described embodiment, although the at-home patients are treated together with the outpatients and inpatients, the present invention is not limited thereto, and can be applied to a cyber-hospital which primarily treats at-home patients.

In the above-described embodiment, the reception server and the communication server are provided in the hospital. However, the present invention is not limited thereto, and the reception server and the communication server may be provided at a site that is different from the hospital, and the at-home medical consultation service of the present invention may be provided through a network.

Further, the doctor terminal and the nurse terminal need not necessarily be provided in the hospital, and the doctor and the nurse may offer an at-home medical service through the network.

In the above-described embodiment, the administration server is provided independently from the reception server. However, the reception server may be provided with an administration database and may include a function for administrating of the hospital.

In the above-described embodiment, the doctor terminal, nurse terminal and waiting room terminal are provided regardless of the medical consultation departments. In a large-scale hospital, however, the terminals are provided by each medical consultation departments, and are selected depending upon the department selected by a patient.

In the above-described embodiment, a single nurse terminal is provided assuming that the nurse terminal is installed in, for example, a nurse center to check the virtual nursing rooms where the persons who need nursing regularly, in the same manner as going around to the sickrooms in a hospital. However the nurse center may be provided with a nurse terminal having a large screen for monitoring the virtual nursing rooms, and the individual consultation rooms with separate nurse terminals to assist the medical consultation by the doctor.

In the above-described embodiment, the nurse call is conducted by either clicking the "NURSE CALL" button provided on the screen displayed on the patient terminal or by pushing the nurse call button connected to the patient terminal. However, either one of these may be employed as a matter of course.

In the above-described embodiment, the video, audio and medical examination sensor data at the patient terminal entered in the nursing room are transmitted to the nurse terminal and/or the doctor terminal when requested from the nurse terminal and/or the doctor terminal. However, the video, audio and medical examination sensor data of the patient terminal entered in the nursing room may be partially or completely continuously transmitted to a particular terminal. For example, by installing a dedicated terminal for monitoring the nursing room in the nurse center, and continuously displaying a portion or all of the video, audio and medical examination sensor data of the patient terminal entered in the nursing room, a deterioration in the condition of a person who requires nursing can be quickly determined.

In this case, the signal level of the medical examination sensors transmitted from the patient terminal entered in the nursing room may be monitored at the terminal, and an alarm may be displayed at the terminal when a preset alarm level is exceeded. Thus, a sudden deterioration in the condition can be dealt with even if the video of the patient terminal in the nursing room is not always monitored at the terminal.

Furthermore, the patient terminal may be provided with a function for monitoring the signal level of the medical examination sensors, and the nurse call may be informed when an alarm level set by the nurse terminal or the doctor terminal is exceeded. Thus, a deterioration in the condition is automatically detected and notified as long as the patient is wearing the medical examination sensor, and the situation even when the patient is not capable of pushing the nurse call button can be dealt with, such that the patient at-home receives the at-home consultation service without anxiety.

In the above-described embodiment, the calling procedure for the patient terminal displayed on the screen of the virtual waiting room or the virtual nursing room is executed by obtaining the click location and comparing the click location with the coordinates of the patient terminals that are displayed to select a callee patient terminal. A map describing a relationship between the clicked location and the corresponding patient terminal may be generated to execute a calling procedure relying on the clickable map.

The above-described embodiment does not describe the accounting procedure of fees for consulting the patient at home. The system, however, processes every medical work by using medical consultation files in the same manner as treating outpatients in general. Therefore, the medical fees for the patients at home can be calculated using the same processing as the ordinary point processing.

Furthermore, the at-home medical consultation system may be connected to banking institutions to automatically charge the medical fees to a bank account.

### Industrial Applicability

As mentioned above, according to the present invention, even when the patient terminal has not been connected to the doctor terminal, a sudden deterioration in the patient's condition can be dealt with, the patient's anxiety for the sickness and sense of isolation are eliminated without increasing the burden on a doctor, and an at-home medical consultation service in an environment similar to when the patient actually visits a hospital is provided.

## Claims

1. An at-home medical consultation system which provides an at-home medical consulting service by connecting a patient terminal having a videophone function to a doctor terminal having a videophone function through a communication line, the at-home medical consultation system comprising:
a reception server for accepting a request for medical consultation from the patient terminal; and
a communication server for providing a bidirectional communication function of video and audio by connecting the patient terminal whose request for medical consultation has been accepted to the doctor terminal; wherein-
the communication server includes a function for setting a virtual waiting room for providing a conversation service by video and audio among the patient terminals; and
the reception server includes a function for connecting the patient terminal whose request for medical consultation has been accepted to the virtual waiting room that has been set.

2. The at-home medical consultation system according to claim 1, wherein the communication server includes a function for connecting a waiting room terminal having a videophone function installed in a waiting room of a hospital to the virtual waiting room that has been set.

3. The at-home medical consultation system according to claim 1 or 2, wherein:
the reception server includes a function for selecting a callee terminal connected to the virtual waiting room based on a location on a screen specified by a pointing device at the caller terminal connected to the virtual waiting room; and
the communication server includes a function for providing a conversation service by video and audio by separately connecting the caller terminal to the callee terminal.

4. The at-home medical consultation system according to in any one of claims 1 to 3, wherein:
the reception server includes a function for accepting a nurse call from the patient terminal and notifying a nurse terminal having a videophone function of the nurse call; and
the communication server includes a function for providing a conversation service by video and audio by connecting the patient terminal whose nurse call has been accepted to the notified nurse terminal.

5. The at-home medical consultation system according to claim 4, wherein the patient terminal includes a nurse call button for transmitting a nurse call to the reception server, and includes a function for automatically logging into the reception server when the nurse call button is pressed to transmit the nurse call.

6. The at-home medical consultation system according to claim 4 or 5, wherein:
the communication server includes a function for setting a virtual nursing room to which is connected the patient terminal used by a person who needs nursing; and
the reception server includes a function for connecting the patient terminal used by the person who needs nursing to the virtual nursing room upon a request from the patient terminal, the doctor terminal or the nurse terminal; and
the communication server includes a function for transmitting the video and audio of the patient terminals connected to the virtual nursing room to the doctor terminal and/or the nurse terminal upon a request from the doctor terminal and/or the nurse terminal.

7. The at-home medical consultation system according to claim 6, wherein:
the reception server includes a function for selecting a patient terminal connected to the virtual nursing room based on a location on a screen specified by the pointing device at the doctor terminal and/or the nurse terminal; and
the communication server includes a function for providing a conversation service by video and audio by separately connecting the doctor terminal and/or the nurse terminal to the selected patient terminal.

8. The at-home medical consultation system according to claim 6 or 7, wherein:
the patient terminal includes medical examination sensors for collecting the data necessary for consulting the patient, and includes a function for obtaining the data from the medical examination sensors and transmitting the data to the communication server;
the communication server includes a function for receiving the transmitted data from the medical examination sensors and transmitting the data to the doctor terminal and/or the nurse terminal; and
the doctor terminal and/or the nurse terminal include a function for receiving the transmitted data from the medical examination sensors and displaying them.

9. The at-home medical consultation system according to claim 8, wherein the patient terminal includes a function for transmitting a portion or all of the signals of the medical examination sensors as audio signals.

10. An at-home medical consultation system which provides an at-home medical consulting service by connecting a patient terminal having a videophone function to a doctor terminal having a videophone function through a communication line, the at-home medical consultation system comprising:
a reception server for accepting a request for medical consultation from the patient terminal; and
a communication server for providing a bidirectional communication function of video and audio by connecting the patient terminal whose request for medical consultation has been accepted to the doctor terminal; wherein
the communication server includes a function for setting a virtual nursing room to which the patient terminal used by a person who needs nursing is connected;
the reception server includes a function for connecting the patient terminal used by the person who needs nursing to the virtual nursing room upon a request from the patient terminal, the doctor terminal or the nurse terminal having the videophone function;
the communication server includes a function for transmitting video and audio of the patient terminals connected to the virtual nursing room to the doctor terminal and/or the nurse terminal upon a request from the doctor terminal and/or the nurse terminal; and
the doctor terminal and/or the nurse terminal includes a function for displaying the transmitted video of the patient terminals that have been connected to the virtual nursing room.

11. The at-home medical consultation system according to claim 10, wherein:
the reception server includes a function for selecting the patient terminal connected to the virtual nursing room based on a location on a screen specified by a pointing device at the doctor terminal and/or the nurse terminal; and
the communication server includes a function for providing a conversation service by video and audio by separately connecting the doctor terminal and/or the nurse terminal to the selected patient terminal.

12. The at-home medical consultation system according to claim 10 or 11, wherein:
the reception server includes a function for accepting the nurse call from the patient terminal and notifying the nurse terminal of the nurse call; and
the communication server includes a function for providing a conversation service by video and audio by connecting the patient terminal whose nurse call has been accepted to the notified nurse terminal.

13. The at-home medical consultation system according to claim 12, wherein the patient terminal includes a nurse call button for transmitting a nurse call to the reception server, and includes a function for automatically logging into the reception server, when the nurse call button is pressed to transmit the nurse call.

14. The at-home medical consultation system according to any one of claims 10 to 13, wherein:
the patient terminal includes medical examination sensors for collecting the data necessary for consulting the patient, and includes a function for obtaining the data from the medical examination sensors and transmitting the data to the communication server;
the communication server includes a function for receiving the transmitted data from the medical examination sensors and transmitting the data to the doctor terminal and/or the nurse terminal; and
the doctor terminal and/or the nurse terminal include a function for receiving the transmitted data from the medical examination sensors and displaying them.

15. The at-home medical consultation system according to claim 14, wherein the patient terminal includes a function for transmitting a portion or all of the signals of the medical examination sensors as voice signals.

16. An at-home medical consultation system which provides an at-home medical consulting service by connecting the patient terminal having a videophone function to the doctor terminal having a videophone function through a communication line, the at-home medical consultation system comprising:
a reception server for accepting a request for medical consultation from the patient terminal; and
a communication server for providing a bidirectional communication function of video and audio by connecting the patient terminal whose request for medical consultation has been accepted to the doctor terminal; wherein
the reception server includes a function for accepting a nurse call from the patient terminal and notifying the nurse terminal having the videophone function of the nurse call; and
the communication server includes a function for providing a conversation service by video and audio by connecting the patient terminal whose nurse call has been accepted to the notified nurse terminal.

17. The at-home medical consultation system according to claim 16, wherein the patient terminal includes a nurse call button for transmitting a nurse call to the reception server, and includes a function for automatically logging into the reception server when the nurse call button is pressed to transmit the nurse call.

18. The at-home medical consultation system according to any one of claims 1 to 17, wherein the reception server includes a function for accepting a request for medication from the patient terminal, a function for requesting the doctor terminal used by a doctor in charge of the patient whose request for medication has been accepted to confirm the medication, and a function for delivering a prescription issued by the doctor terminal in response to the request to a pharmacy terminal used by a pharmacy that offers home delivery service of medicine.

19. The at-home medical consultation system according to any one of claims 1 to 18, wherein the reception server includes a function for accepting a request for counseling from the patient terminal, a function for receiving counseling sentences from the patient terminal whose request for counseling has been accepted, a function for transmitting the received counseling sentences to the doctor terminal used by a doctor in charge, a function for receiving reply sentences from the doctor terminal in response to the transmitted counseling sentences, and a function for transmitting the received reply sentences to the patient terminal whose request for counseling has been accepted.

20. The at-home medical consultation system according to any one of claims 1 to 19 , wherein the reception server includes a function for accepting a reservation for visit consultation from the patient terminal.

21. An at-home medical consultation method which provides an at-home medical consulting service by connecting a patient terminal having a videophone function to a doctor terminal having a videophone function through a communication line, the at-home medical consultation method comprising:
a step of accepting a request for medical consultation from the patient terminal; and
a step of providing a bidirectional communication function of video and audio by connecting the patient terminal whose request for medical consultation has been accepted to the doctor terminal; wherein
the step of providing the bidirectional communication of video and audio includes:
a step of setting a virtual waiting room for providing a conversation service by video and audio among the accepted patient terminals; and
the step of accepting the request for medical consultation includes a step of connecting the patient terminal whose request for medical consultation has been accepted to the virtual waiting room that has been set.

22. The at-home medical consultation method according to claim 21, wherein the step of providing the bidirectional communication of video and audio includes a step of connecting a waiting room terminal having a videophone function installed in a waiting room of a hospital to the virtual waiting room that has been set.

23. The at-home medical consultation method according to claim 21 or 22, wherein:
the step of accepting the request for medical consultation includes a step of selecting a callee terminal connected to the virtual waiting room based on a location on a screen specified by a pointing device at the caller terminal connected to the virtual waiting room; and
the step of providing the bidirectional communication of video and audio includes a step of providing a conversation service by video and audio by separately connecting the caller terminal to the callee terminal.

24. The at-home medical consultation method according to any one of claims 21 to 23, wherein:
the step of accepting the request for medical consultation includes a step of accepting a nurse call from the patient terminal and notifying a nurse terminal having a videophone function of the nurse call; and
the step of providing the bidirectional communication of video and audio includes a step of providing a conversation service by video and audio by connecting the patient terminal whose nurse call has been accepted to the notified nurse terminal.

25. The at-home medical consultation method according to claim 24, wherein:
the step of providing the bidirectional communication of video and audio includes a step of setting a virtual nursing room to which is connected the patient terminal used by a person who needs nursing;
the step of accepting the request for medical consultation includes a step of connecting the patient terminal used by the person who needs nursing to the virtual nursing room upon a request from the patient terminal, the doctor terminal or the nurse terminal; and
the step of providing the bidirectional communication of video and audio includes a step of transmitting the video and audio of the patient terminals connected to the virtual nursing room to the doctor terminal and/or the nurse terminal upon a request from the doctor terminal and/or the nurse terminal.

26. The at-home medical consultation method according to claim 25, wherein:
the step of accepting the request for medical consultation includes a step of selecting a patient terminal connected to the virtual nursing room based on a location on a screen specified by the pointing device at the doctor terminal and/or the nurse terminal; and
the step of providing the bidirectional communication of video and audio includes a step of providing a conversation service by video and audio by separately connecting the doctor terminal and/or the nurse terminal to the selected patient terminal.

27. The at-home medical consultation method according to claim 25 or 26, wherein the step of providing the bidirectional communication of video and audio includes a step of receiving the data obtained from the medical examination sensors provided at the patient terminal and transmitting them to the doctor terminal and/or the nurse terminal.

28. An at-home medical consultation method which provides an at-home medical consulting service by connecting a patient terminal having a videophone function to a doctor terminal having a videophone function through a communication line, the at-home medical consultation method comprising:
a step of accepting a request for medical consultation from the patient terminal; and
a step of providing a bidirectional communication function of video and audio by connecting the patient terminal whose request for medical consultation has been accepted to the doctor terminal; wherein
the step of providing the bidirectional communication of video and audio includes a step of setting a virtual nursing room to which is connected the patient terminal used by the person who needs nursing;
the step of accepting the request for consultation includes a step of connecting the patient terminal used by the person who needs nursing to the virtual nursing room upon a request from the patient terminal, the doctor terminal or the nurse terminal having the videophone function; and
the step of providing the bidirectional communication of video and audio includes a step of transmitting the video and audio of the patient terminals connected to the virtual nursing room to the doctor terminal and/or the nurse terminal upon a request from the doctor terminal and/or the nurse terminal.

29. The at-home medical consultation method according to claim 28, wherein:
the step of accepting the request for medical consultation includes a step of selecting the patient terminal connected to the virtual nursing room based on a location on a screen specified by a pointing device at the doctor terminal and/or the nurse terminal; and
the step of providing the bidirectional communication of video and audio includes a step of providing a conversation service by videos and audios by separately connecting the doctor terminal and/or the nurse terminal to the selected patient terminal.

30. The at-home medical consultation method according to claim 28 or 29, wherein the step of accepting a request for medical consultation includes a step of accepting the nurse call from the patient terminal and notifying the nurse terminal of the nurse call; and
the step of providing the bidirectional communication of video and audio includes a step of providing a conversation service by video and audio by connecting the patient terminal whose nurse call has been accepted to the notified nurse terminal.

31. The at-home medical consultation method according to any one of claims 28 to 30, wherein the step of providing the bidirectional communication of video and audio includes a step of receiving the data obtained by the medical examination sensors provided at the patient terminal and transmitting the data to the doctor terminal and/or the nurse terminal.

32. An at-home medical consultation method which provides an at-home medical consulting service by connecting a patient terminal having a videophone function to a doctor terminal having a videophone function through a communication line, the at-home medical consultation method comprising:
a step of accepting a request for medical consultation from the patient terminal; and
a step of providing a bidirectional communication function of video and audio by connecting the patient terminal whose request for medical consultation has been accepted to the doctor terminal; wherein
the step of accepting the request for medical consultation includes a step of accepting a nurse call from the patient terminal and notifying a nurse terminal having a videophone function of the nurse call; and
the step of providing the bidirectional communication by video and audio includes a step of providing a conversation service by video and audio by connecting the patient terminal whose nurse call has been accepted to the notified nurse terminal.

33. The at-home medical consultation method according to any one of claims 21 to 32, wherein the step of accepting a request for medical consultation includes a step of accepting a request for a medication from the patient terminal, a step of requesting the doctor terminal used by the doctor in charge of the patient whose request for medication has been accepted to confirm the medication, and a step of delivering a prescription issued by the doctor terminal in response to the request to a pharmacy terminal used by a pharmacy that offers a home delivery service of medicine.

34. The at-home medical consultation method according to any one of claims 21 to 33 , wherein the step of accepting the request for medical consultation includes a step of accepting a request for counseling from the patient terminal, a step of receiving counseling sentences from the patient terminal whose request for counseling has been accepted, a step of transmitting the received counseling sentences to the doctor terminal used by a doctor in charge, a step of receiving reply sentences from the doctor terminal in response to the transmitted counseling sentences, and a step of transmitting the received reply sentences to the patient terminal whose request for counseling has been accepted.

35. The at-home medical consultation method according to any one of claims 21 to 34, wherein the step of accepting a request for medical consultation includes a step of accepting a reservation of visit consultation from the patient terminal.

36. An at-home medical consultation program for executing, by using a computer, the at-home medical consultation method described in any one of claims 21 to 35.
